(19) Europäisches Patentamt
European Patent Office
Office européen des brevets

(11) EP 4 471 016 A1

(12) EUROPEAN PATENT APPLICATION
published in accordance with Art. 153(4) EPC

(43) Date of publication:
**04.12.2024 Bulletin 2024/49**

(21) Application number: **23746231.2**

(22) Date of filing: **19.01.2023**

(51) International Patent Classification (IPC):
***C07D 279/20*** *(2006.01)* ***A61K 31/5415*** *(2006.01)*
***A61P 35/00*** *(2006.01)* ***A61P 3/00*** *(2006.01)*
***A61P 9/00*** *(2006.01)* ***A61P 25/00*** *(2006.01)*

(52) Cooperative Patent Classification (CPC):
**A61K 31/5415; A61P 3/00; A61P 9/00; A61P 25/00; A61P 35/00; C07D 279/20**

(86) International application number:
**PCT/CN2023/073055**

(87) International publication number:
**WO 2023/143350 (03.08.2023 Gazette 2023/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.01.2022 CN 202210108728**
**28.01.2022 CN 202210108746**

(71) Applicant: **Chengdu Henghao Innovative Science and Technology Co., Ltd.**
**Chengdu, Sichuan 610000 (CN)**

(72) Inventors:
- **YU, Zhou**
  **Chengdu, Sichuan 610000 (CN)**
- **HE, Peng**
  **Chengdu, Sichuan 610000 (CN)**
- **DONG, Guangxin**
  **Chengdu, Sichuan 610000 (CN)**
- **LI, Yi**
  **Chengdu, Sichuan 610000 (CN)**
- **ZHANG, Rui**
  **Chengdu, Sichuan 610000 (CN)**
- **DENG, Ta**
  **Chengdu, Sichuan 610000 (CN)**
- **YE, Qijun**
  **Chengdu, Sichuan 610000 (CN)**
- **HUANG, Pei**
  **Chengdu, Sichuan 610000 (CN)**
- **LI, Shan**
  **Chengdu, Sichuan 610000 (CN)**
- **LI, Bogang**
  **Chengdu, Sichuan 610000 (CN)**

(74) Representative: **Eisenführ Speiser**
**Patentanwälte Rechtsanwälte PartGmbB**
**Postfach 10 60 78**
**28060 Bremen (DE)**

# (54) CRYSTAL FORM AND SALT OF PHENOTHIAZINE COMPOUND, PREPARATION METHOD THEREFOR AND USE THEREOF

(57) Provided are a crystal form and salt of a phenothiazine compound, a preparation method therefor and a use thereof. A hydrochloride of the phenothiazine compound and a crystal thereof can be used as ferroptosis inhibitors, and the hydrochloride and the crystal thereof have relatively good solubility, stability, oral absorption bioavailability, and druggability.



Processed by Luminess, 75001 PARIS (FR)

## Description

This application claims the priority of Chinese Patent Application No. 202210108728.0 and 202210108746.9, filed with the China National Intellectual Property Administration on 28 January 2022, which are hereby incorporated by reference in their entirety.

## FIELD

The present disclosure belongs to the field of medicinal chemistry, and in particular relates to a crystal form of a medicinal phenothiazine compound, a salt, a preparation method therefor and use thereof.

## BACKGROUND

Ferroptosis is iron-dependent programmed cell death driven by lipid peroxidation. Morphologically, ferroptosis mainly manifests as reduced mitochondrial volume, increased mitochondrial membrane density, reduction or disappearance of mitochondrial cristae and rupture of the outer mitochondrial membrane, whereas the size of the nucleus is normal. These are main morphological characteristics of ferroptosis that are distinct from apoptosis, necrosis, and autophagy. Biochemical features in ferroptosis involve accumulation of intracellular iron and reactive oxygen species (ROS), activation of mitogen-activated protein kinase (MAPK) signaling, inhibition of cystine/glutamate transporter protein system, increased NADPH oxidation, etc.

More and more researches confirm that cellular ferroptosis is tightly associated with many conditions, disorders and diseases. It was first identified by Dixon et al, in 2012 (Scott J Dixon et al. Cell. 2012 May 25; 149(5): 1060-72.) that this mode of cell death is associated with small molecule-inducedtumor cell death involving RAS. Recent studies have further demonstrated the role of ferroptosis in cancer, organ injury, and degenerative diseases (Xuejun Jiang et al. Nat Rev Mol Cell Biol. 2021 Apr; 22(4):266-282). Specifically, it is involved in the occurrence and development of various cancers, neurodegenerative diseases, cardiovascular and cerebrovascular diseases, immune-related diseases, liver and kidney failure, inflammation, and metabolic diseases. In particular, it plays an important role in Alzheimer's disease, Parkinson's disease, tumors, stroke, ischemia-reperfusion injury, atherosclerosis, liver and kidney failure, inflammation, diabetic complications, etc. Usually, the stimulation or inhibition on ferroptosis can suppress the occurrence and development of related diseases. Therefore, it is believed that the ferroptosis inhibitor is a potential medicament for the treatment of these diseases.

Compound 2-(1-(4-(4-methylpiperazin-1-yl)phenyl)ethyl)-10R-phenothiazine and a preparation method therefor have been disclosed in international application No. WO2019205854A1, which exhibits excellent inhibitory activity on ferroptosis. However, there is no study on its crystal form in the prior art.

It is a common phenomenon that a drug has polymorphic forms in drug development, which is an important factor affecting the quality of the drug. Different crystal forms have different solubility, stability, hygroscopicity and bioavailability, thereby directly affecting the quality, and absorption behavior in the human body of the pharmaceutical formulation comprising the drug, and ultimately affecting the benefit ratio of the therapeutic effect and side effect produced by the formulation in the human body. Therefore, it is necessary to study the crystal form of a compound to develop a new crystal form with high purity, good solubility and stability as well as high bioavailability to maximize the effectiveness of the drug, which is of great importance in drug development.

## SUMMARY

An object of the present disclosure is to provide various crystal forms of 2-(1-(4-(4-methylpiperazin-1-yl)phenyl)ethyl)-10H-phenothiazine, in particular various crystal forms of its hydrochloride salt, which have improved druggability, such as stability, solubility, hygroscopicity, and bioavailability.

Another object of the present disclosure is to provide a pharmaceutical composition comprising the above crystal form, a preparation method therefor and use thereof.

In order to achieve the above objects, the present disclosure provides the following technical solutions:
In one or more embodiments, provided is a crystal Form A of 2-(1-(4-(4-methylpiperazin-1-yl)phenyl)ethyl)-10H-phenothiazine hydrochloride, wherein the X-ray powder diffraction pattern with Cu-K$\alpha$ radiation of the crystal Form A comprises characteristic peaks at 2$\theta$ of 10.68$\pm$0.2°, 14.36$\pm$0.2°, 18.57$\pm$0.2°, 21.08$\pm$0.2°, 22.14$\pm$0.2°, 23.40$\pm$0.2°, and 29.03$\pm$0.2°.

In one or more embodiments, the X-ray powder diffraction pattern with Cu-K$\alpha$ radiation of the crystal Form A comprises characteristic peaks at 2$\theta$ of 10.68$\pm$0.2°, 14.36$\pm$0.2°, 17.84$\pm$0.2°, 18.57$\pm$0.2°, 21.08$\pm$0.2°, 22.14$\pm$0.2°, 23.40$\pm$0.2°, 27.50$\pm$0.2°, and 29.03$\pm$0.2°.

In one or more embodiments, the X-ray powder diffraction pattern with Cu-K$\alpha$ radiation of the crystal Form A

comprises characteristic peaks at 2θ of 10.68±0.2°, 14.36±0.2°, 16.54±0.2°, 17.84±0.2°, 18.57±0.2°, 20.89±0.2°, 21.08±0.2°, 22.14±0.2°, 22.92±0.2°, 23.40±0.2°, 25.88±0.2°, 27.50±0.2°, and 29.03±0.2°.

In one or more embodiments, the X-ray powder diffraction pattern with Cu-Kα radiation of the crystal Form A comprises characteristic peaks at 2θ of 10.68±0.2°, 12.63±0.2°, 14.36±0.2°, 16.06±0.2°, 16.54±0.2°, 17.84±0.2°, 18.57±0.2°, 20.89±0.2°, 21.08±0.2°, 22.14±0.2°, 22.92±0.2°, 23.40±0.2°, 25.11±0.2°, 25.51±0.2°, 25.88±0.2°, 27.50±0.2°, 28.54±0.2°, 29.03±0.2°, and 33.55±0.2°.

In one or more embodiments, the X-ray powder diffraction pattern with Cu-Kα radiation of the crystal Form A at 2θ is shown in FIG. 1.

In one or more embodiments, provided is a crystal Form B of 2-(1-(4-(4-methylpiperazin-1-yl)phenyl)ethyl)-10H-phenothiazine hydrochloride, wherein the X-ray powder diffraction pattern with Cu-Kα radiation of the crystal Form B comprises characteristic peaks at 2θ of 16.46±0.2°, and 22.01±0.2°.

In one or more embodiments, the X-ray powder diffraction pattern with Cu-Kα radiation of the crystal Form B comprises characteristic peaks at 2θ of 16.46±0.2°, 22.01±0.2°, 27.62±0.2°, 28.91±0.2°, and 33.41±0.2°.

In one or more embodiments, the X-ray powder diffraction pattern with Cu-Kα radiation of the crystal Form B comprises characteristic peaks at 2θ of 5.41±0.2°, 10.92±0.2°, 16.46±0.2°, 22.01±0.2°, 27.62±0.2°, 28.91±0.2°, and 33.41±0.2°.

In one or more embodiments, the X-ray powder diffraction pattern with Cu-Kα radiation of the crystal Form B at 20 is shown in FIG. 3.

In one or more embodiments, provided is a pharmaceutical composition comprising the hydrochloride salt crystal Form A, or the hydrochloride salt crystal Form B, and a pharmaceutically acceptable carrier.

In one or more embodiments, provided is use of the hydrochloride salt crystal Form A, the hydrochloride salt crystal Form B, or the pharmaceutical composition in the manufacture of a medicament for preventing and/or treating a disease selected from the group consisting of a cancer, organ damage, and degenerative disease.

In one or more embodiments, provided is use of the hydrochloride salt crystal Form A, the hydrochloride salt crystal Form B, or the pharmaceutical composition in the manufacture of a medicament for preventing and/or treating a disease selected from the group consisting of a cancer, neurodegenerative disease, cardiovascular and cerebrovascular disease, immune-related disease, liver and kidney failure, inflammation, and metabolic diseases.

In one or more embodiments, the disease is selected from, but not limited to, the group consisting of a cancer, Alzheimer's disease, Parkinson's disease, multiple sclerosis, Huntington's chorea, amyotrophic lateral sclerosis, stroke, ischemia-reperfusion injury, atherosclerosis, immune-related disease, liver and kidney failure, inflammation, diabetes, and diabetic complications.

In one or more embodiments, the stroke is hemorrhagic stroke and/or ischemic stroke. The ischemic stroke is also known as cerebral infarction.

In one or more embodiments, provided is use of the hydrochloride salt crystal Form A, the hydrochloride salt crystal Form B, or the pharmaceutical composition in the manufacture of a ferroptosis inhibitor.

In one or more embodiments, provided is a method for producing the hydrochloride salt crystal Form A according to the present disclosure, comprising dispersing the compound of 2-(1-(4-(4-methylpiperazin-1-yl)phenyl)ethyl)-10H-phenothiazine in methanol at 10°C to 40°C, followed by dropwise adding a mixed solution of concentrated hydrochloric acid and methanol, stirring for crystallization, filtering, washing a filter cake with methanol, and drying the filter cake under vacuum to obtain a crystal substance.

Preferably, a weight ratio of the methanol as a solvent to the compound of 2-(1-(4-(4-methylpiperazin-1-yl) phenyl)ethyl)-10H-phenothiazine is 5:1-15:1, for example, 5:1, 5.5:1, 6:1, 6.5:1, 7:1, 7.5:1, 8:1, 8.5:1, 9:1, 9.5:1, 10:1, 10.46:1, 11:1, 11.5:1, 12:1, 12.5:1, 13:1, 13.5:1, 14:1, 14.5:1, or 15:1.

Preferably, a weight ratio of the compound of 2-(1-(4-(4-methylpiperazin-1-yl)phenyl)ethyl)-10H-phenothiazine to the concentrated hydrochloric acid is 1:1-8:1, for example, 1:1, 1.5:1, 2:1, 2.5:1, 3:1, 3.5:1, 4:1, 4.15:1, 4.5:1, 5:1, 5.5:1, 6:1, 6.5:1, 7:1, 7.5:1, or 8:1.

Preferably, the stirring for crystallization is performed for 1h to 8h, for example, 1h, 2h, 3h, 4h, 5h, 6h, 7h or 8h.

In one or more embodiments, provided is a method for producing the hydrochloride salt crystal Form A, comprising dispersing a compound of 2-(1-(4-(4-methylpiperazin-1-yl)phenyl)ethyl)-10R-phenothiazine in acetone and water at 10°C to 40°C, followed by dropwise adding a mixed solution of concentrated hydrochloric acid, acetone and water, stirring for crystallization, filtering, washing a filter cake with acetone, and drying the filter cake under vacuum to obtain a crystal substance.

Preferably, a weight ratio of the acetone and water as solvent to the compound of 2-(1-(4-(4-methylpiperazin-1-yl) phenyl)ethyl)-10H-phenothiazine is (1-8):(0.5-3):(0.5-3), preferably (4-6):(0.5-1):(0.5-1); for example 4:1:1, 4.74:1:1, or 5:1:1.

Preferably, a weight ratio of the compound of 2-(1-(4-(4-methylpiperazin-1-yl)phenyl)ethyl)-10H-phenothiazine to the concentrated hydrochloric acid is 1:1-8:1, for example, 1:1, 1.5:1, 2:1, 2.5:1, 3:1, 3.66:1, 4:1, 4.5:1, 5:1, 5.5:1, 6:1, 6.5:1, 7:1, 7.5:1, or 8:1.

Preferably, the stirring for crystallization is performed for 1h to 8h, preferably for 2h to 5h.

The mass fraction of the concentrated hydrochloric acid in the preparation method above is not less than 20%, such as 20%, 25%, 30%, 35%, 36%, 37%, 38%, 39%, or 40%.

The term "organ injury" used herein may refer to any damage, impairment, reduction or loss of one or more functions of an organ or tissue associated with the organ. Injuries may include, but are not limited to, changes in organ tissue or structure in the form of damage or change from normal conditions, for example, the development of areas of tissue necrosis, or disruption or loss of integrity of cellular or tissue structures, or an abnormal aggregation of cellular matter or debris from for example, cell inflammation processes or cell apoptosis. It should be appreciated that different organ and tissue types may present different injury pathologies. An organ injury may also lead to the pathogenesis of a condition or disease associated with the injury, i.e. a condition associated with the organ injury.

A molar ratio of 2-(1-(4-(4-methylpiperazin-1-yl)phenyl)ethyl)-10R-phenothiazine to hydrochloric acid in the hydrochloride salt crystal form of the present disclosure is 1:(1±0.5); for example1:(1±0.4), 1:(1±0.2) or 1:(1±0.1), such as 1:1.

The crystal Form A of 2-(1-(4-(4-methylpiperazin-1-yl)phenyl)ethyl)-10H-phenothiazine hydrochloride in the present disclosure is also referred to as crystal form A; and the crystal Form B of 2-(1-(4-(4-methylpiperazin-1-yl)phenyl) ethyl)-10H-phenothiazine hydrochloride in the present disclosure is also referred to as crystal Form B.

Compared to the prior art, the present disclosure can achieve the following beneficial effects:

1. In the present disclosure, after a large number of experimental investigations, two new crystal forms of the compound are obtained which have a significant improvement in physicochemical properties compared to the free compound.

(1) The crystal Form A is of high purity. For example, in an embodiment of the present disclosure, the purity thereof reaches 99.76%. The crystal Form A exhibits a 10,000-fold increase in solubility in water compared to the free compound, and maintains good stability at the same time. For example, in an embodiment of the present disclosure, the crystal Form A is unchanged under grinding, which shows a good mechanical stability. In another embodiment of the present disclosure, the melting point of the crystal Form A is about 265.31°C, indicating that it has good thermal stability. In another embodiment of the present disclosure, the crystal Form A still retains the same crystal form and its purity is substantially unchanged under accelerated and stable conditions. In another embodiment of the present disclosure, after crystal Form A is slurried in different solvents and magnetically stirred, no crystal transformation occurs. Furthermore, the crystal Form A also has a significant improvement in bioavailability compared to the free compound.

(2) The crystal Form B also has unexpected beneficial effects in solubility compared to the free compound. For example, in an embodiment of the present disclosure, the solubility of the crystal Form B in water is increased by 1000 times compared to the free compound.

2. The crystal Form A has better physicochemical properties compared to the crystal Form B in the present disclosure.

In terms of hygroscopicity, the crystal Form A has a vapor sorption of only 0.36% at 25°C/80% RH, indicating that it is slightly hygroscopic, which is superior to the crystal form B. In terms of solubility, crystal Form A has a better solubility than crystal Form B in both water and hydrochloric acid, e.g., in water and a hydrochloric acid solution with a pH value of 1.0, the solubility of crystal Form A is greater than that of crystal Form B by more than 10 times. In addition, in terms of stability, the crystal Form A with better solubility instead has better stability. For example, in an embodiment of the present disclosure, crystal Form A is unchanged in crystal form under accelerated and stabilized conditions, whereas crystal Form B starts to transform to crystal Form A on day 2 at room temperature. In terms of stability, after subjecting the crystal Form A to a long-term condition of 25°C/60% RH and an accelerated condition of 40°C/75% RH for one week, its purity is essentially unchanged, its chemical stability is good and no changes in crystal form are observed in the samples. After subjecting the crystal Form A to a condition of RT-75% RH and RT-97% RH for one week, no changes in crystal form are observed. After grinding, the crystal Form A is unchanged in crystal form. In addition, under accelerated conditions (40°C ±2°C,75%RH±10%RH, 6 months), the total impurity content of the crystal Form A is significantly lower than that of crystal form B, and its stability is better than that of crystal form B.

Another object of the present disclosure is to provide a variety of pharmaceutically acceptable salts of the compound 2-(1-(4-(4-methylpiperazin-1-yl)phenyl)ethyl)-10H-phenothiazine, which show a significant improvement over the free compound in terms of both solubility and stability.

Another object of the present disclosure is to provide a method for preparing the salts above and use thereof.

In view of this, the present disclosure provides the following specific technical solutions:

In one or more embodiments, provided are 2-(1-(4-(4-methylpiperazin-1-yl)phenyl)ethyl)-10H-phenothiazine hydrochloride, 2-(1-(4-(4-methylpiperazin-1-yl)phenyl)ethyl)-10H-phenothiazine mesylate, 2-(1-(4-(4-methylpiperazin-1-yl)phenyl)ethyl)-10H-phenothiazine esylate, 2-(1-(4-(4-methylpiperazin-1-yl)phenyl)ethyl)-10H-phenothiazine p-toluenesulfonate, 2-(1-(4-(4-methylpiperazin-1-yl)phenyl)ethyl)-10H-phenothiazine citrate, 2-(1-(4-(4-methylpiperazin-1-yl)phenyl)ethyl)-10H-phenothiazine fumarate, 2-(1-(4-(4-methylpiperazin-1-yl)phenyl)ethyl)-10H-phenothiazine maleate, 2-(1-(4-(4-methylpiperazin-1-yl)phenyl)ethyl)-10H-phenothiazine tartrate, 2-(1-(4-(4-methylpiperazin-1-yl)phenyl)ethyl)-10H-phenothiazine hydrobromide, 2-(1-(4-(4-methylpiperazin-1-yl)phenyl)ethyl)-10H-phenothiazine oxalate, 2-(1-(4-(4-methylpiperazin-1-yl)phenyl)ethyl)-10H-phenothiazine phosphate, 2-(1-(4-(4-methylpiperazin-1-yl)phenyl)ethyl)-10H-phenothiazine sulphate, 2-(1-(4-(4-methylpiperazin-1-yl)phenyl)ethyl)-10H-phenothiazine acetate, 2-(1-(4-(4-methylpiperazin-1-yl)phenyl)ethyl)-10H-phenothiazine propionate, 2-(1-(4-(4-methylpiperazin-1-yl)phenyl)ethyl)-10H-phenothiazine perchlorate, 2-(1-(4-(4-methylpiperazin-1-yl)phenyl)ethyl)-10H-phenothiazine malate, 2-(1-(4-(4-methylpiperazin-1-yl)phenyl)ethyl)-10H-phenothiazine salicylate, 2-(1-(4-(4-methylpiperazin-1-yl)phenyl)ethyl)-10H-phenothiazine mandelate, 2-(1 -(4-(4-methylpiperazin-1-yl)phenyl)ethyl)-10H-phenothiazine lactate; and 2-(1-(4-(4-methylpiperazin-1-yl)phenyl)ethyl)-10H-phenothiazine succinate.

In one or more embodiments, provided is 2-(1-(4-(4-methylpiperazin-1-yl)phenyl)ethyl)-10H-phenothiazine hydrochloride.

In one or more embodiments, a molar ratio of 2-(1-(4-(4-methylpiperazin-1-yl)phenyl)ethyl)-10H-phenothiazine to hydrochloric acid in the 2-(1-(4-(4-methylpiperazin-1-yl)phenyl)ethyl)-10H-phenothiazine hydrochloride is 1:(0.5-2); for example 1:1, or 1:2.

A molar ratio of the compound of 2-(1-(4-(4-methylpiperazin-1-yl)phenyl)ethyl)-10R-phenothiazine to an acid in the salts of the present disclosure all fluctuate appropriately within an acceptable range.

For example, in one or more embodiments, the molar ratio of 2-(1-(4-(4-methylpiperazin-1-yl)phenyl)ethyl)-10H-phenothiazine to an acid could be 1:(2$\pm$0.4), for example, 1:(2$\pm$0.3), 1:(2$\pm$0.2), 1:(2$\pm$0.1), or 1:2. The acid is selected from the group consisting of hydrochloric acid, methanesulfonic acid, ethanesulfonic acid, p-toluenesulfonic acid, citric acid, fumaric acid, maleic acid, tartaric acid, hydrobromic acid, oxalic acid, phosphoric acid, sulfuric acid, acetic acid, propionic acid, perchloric acid, malic acid, salicylic acid, mandelic acid, lactic acid and succinic acid.

In one or more embodiments, the molar ratio of 2-(1-(4-(4-methylpiperazin-1-yl)phenyl)ethyl)-10H-phenothiazine to an acid is 1:(1$\pm$0.2), for example, 1:(1$\pm$0.15), 1:(1$\pm$0.1), 1:(1$\pm$0.05), or 1:1. The acid is selected from the group consisting of hydrochloric acid, methanesulfonic acid, ethanesulfonic acid, p-toluenesulfonic acid, citric acid, fumaric acid, maleic acid, tartaric acid, hydrobromic acid, oxalic acid, phosphoric acid, sulfuric acid, acetic acid, propionic acid, perchloric acid, malic acid, salicylic acid, mandelic acid, lactic acid and succinic acid.

In one or more embodiments, provided is a method for producing the salt of 2-(1-(4-(4-methylpiperazin-1-yl)phenyl)ethyl)-10H-phenothiazine, comprising performing a reaction of 2-(1-(4-(4-methylpiperazin-1-yl)phenyl)ethyl)-10R-phenothiazine and an acid to form a salt.

Preferably, the reaction is carried out in water and/or an organic solvent, and the solvent is selected from the group consisting of a ketone with 2 to 6 carbon atoms, ethyl acetate, lower fatty alcohol, tetrahydrofuran and a combination thereof. The lower fatty alcohol refers to an alcohol containing 1-8 carbon atoms.

Further, the ketone with 2 to 6 carbon atoms is preferably acetone.

Still further, the lower fatty alcohol is optionally selected from the group consisting of methanol, ethanol, propanol and isopropanol.

Preferably, the reaction is carried out in a solvent selected from the group consisting of acetone, methanol, ethanol, and a combination thereof.

Preferably, the acid is selected from the group consisting of hydrochloric acid, HCl, methanesulfonic acid, ethanesulfonic acid, p-toluenesulfonic acid, citric acid, fumaric acid, maleic acid, and tartaric acid, and more preferably hydrochloric acid. The hydrochloric acid refers to an aqueous solution of HCl, and HCl refers to HCl gas.

In one or more embodiments, provided is a method for producing 2-(1 -(4-(4-methylpiperazin-1-yl)phenyl)ethyl)-10H-phenothiazine hydrochloride, comprising reacting 2-(1-(4-(4-methylpiperazin-1-yl)phenyl)ethyl)-10H-phenothiazine with hydrochloric acid in acetone, ethanol, isopropanol or tetrahydrofuran, stirring, and filtering.

In one or more embodiments, provided is a pharmaceutical composition comprising the salt of 2-(1-(4-(4-methylpiperazin-1-yl)phenyl)ethyl)-10R-phenothiazine and one or more pharmaceutically acceptable carriers and/or diluents; preferably, the salt is 2-(1-(4-(4-methylpiperazin-1-yl)phenyl)ethyl)-10H-phenothiazine hydrochloride.

In one or more embodiments, provided is use of the salt of 2-(1-(4-(4-methylpiperazin-1-yl)phenyl)ethyl)-10H-phenothiazine, or the pharmaceutical composition comprising the same in the manufacture of a medicament for preventing and/or treating a disease selected from the group consisting of a cancer, organ damage and degenerative disease.

In one or more embodiments, provided is use of the salt of 2-(1-(4-(4-methylpiperazin-1-yl)phenyl)ethyl)-10H-phenothiazine, or the pharmaceutical composition comprising the same in the manufacture of a medicament for preventing and/or treating a disease selected from the group consisting of a cancer, neurodegenerative disease, cardiovascular and cerebrovascular disease, immune-related disease, liver and kidney failure, inflammation and metabolic disease.

In one or more embodiments, the disease is selected from the group consisting of a cancer, Alzheimer's disease, Parkinson's disease, multiple sclerosis, Huntington's chorea, amyotrophic lateral sclerosis, stroke, ischemia-reperfusion injury, immune-related disease, liver and kidney failure, inflammation, atherosclerosis, diabetes, and diabetic complications.

In one or more embodiments, the stroke is hemorrhagic stroke and/or ischemic stroke. The ischemic stroke is also known as cerebral infarction.

Compared to the prior art, the present disclosure achieves the following beneficial effects:

1. 2-(1-(4-(4-methylpiperazin-1-yl)phenyl)ethyl)-10H-phenothiazine hydrochloride has improved solubility, stability, and hygroscopicity compared to that of the corresponding free compound.
The solubility of the 2-(1-(4-(4-methylpiperazin-1-yl)phenyl)ethyl)-10H-phenothiazine hydrochloride is significantly improved compared to the corresponding free compound. For example, in an embodiment of the present disclosure, the solubility in water of the hydrochloride salt is improved by about 7700 times compared to that of the corresponding free compound. In addition, other salts, such as citrate salt of the 2-(1-(4-(4-methylpiperazin-1-yl)phenyl)ethyl)-10H-phenothiazine, are also significantly more soluble than the corresponding free compound. The stability of the hydrochloride salt is greatly improved compared to the corresponding free compound. For example, in an embodiment of the present disclosure, the stability of the hydrochloride salt is superior to that of the corresponding free compound under high temperatures, high humidity, and illumination conditions. The total impurity content in the hydrochloride salt is significantly lower. Furthermore, the hydrochloride salt has lower hygroscopicity compared to the corresponding free compound.

2. 2-(1-(4-(4-methylpiperazin-1-yl)phenyl)ethyl)-10H-phenothiazine hydrochloride shows significantly improved performance compared to other salts.

In terms of solubility, the hydrochloride salt has better solubility compared to other salts. For example, in an embodiment of the present disclosure, the hydrochloride salt has a 7-fold increase in the solubility compared to citrate salt with a good solubility, and a more than 35-fold increase in the solubility compared to maleate and fumarate salts. In terms of stability, the hydrochloride salt has a better stability compared to other salts. For example, in an embodiment of the present disclosure, under high temperature, high humidity and illumination conditions, the hydrochloride salt has a better stability than that of other salts, and its total impurity content is significantly lower than that of other salts. In another embodiment, the color of the hydrochloride salt in a hydrochloric acid solution of pH 1.0 is unchanged, whereas the color of other salts, such as citrate, fumarate, and maleate salts, are significantly changed, indicating that hydrochloride salt has a better stability. In terms of hygroscopicity, the hygroscopicity of the hydrochloride salt is lower than that of other salts. In addition, during the preparation process, the hydrochloride salt shows a good solidification property, while other salts such as mesylate and p-toluenesulfonate salts tend to be a viscous paste.

## BRIEF DESCRIPTION OF DRAWINGS

FIG. 1 is the XRPD pattern of the crystal Form A prepared in Example 1.

FIG. 2 is the XRPD pattern of the crystal Form A prepared in Example 2.

FIG. 3 is the XRPD pattern of the crystal Form B.

FIG. 4 shows the result of purity detection of crystal Form A.

FIG. 5 is the $^1$HNMR spectra of the crystal Form A.

FIG. 6 is the TGA profile of the crystal Form A.

FIG. 7 is the DSC pattern of the crystal Form A.

FIG. 8 is the XRPD pattern of the crystal Form A in stability test.

FIG. 9 is the XRPD pattern of the crystal Form B in stability test.

FIG. 10 is the $^{1}$HNMR spectra of 2-(1-(4-(4-methylpiperazin-1-yl)phenyl)ethyl)-10H-phenothiazine hydrochloride.

FIG. 11 is the $^{13}$CNMR spectra of 2-(1-(4-(4-methylpiperazin-1-yl)phenyl)ethyl)-10H-phenothiazine hydrochloride.

FIG. 12 is the $^{1}$HNMR spectra of 2-(1-(4-(4-methylpiperazin-1-yl)phenyl)ethyl)-10H-phenothiazine citrate.

FIG. 13 is the $^{1}$HNMR spectra of 2-(1-(4-(4-methylpiperazin-1-yl)phenyl)ethyl)-10H-phenothiazine fumarate.

FIG. 14 is the $^{1}$HNMR spectra of 2-(1-(4-(4-methylpiperazin-1-yl)phenyl)ethyl)-10H-phenothiazine maleate.

## DETAILED DESCRIPTION

The specific synthesis of 2-(1-(4-(4-methylpiperazin-1-yl)phenyl)ethyl)-10H-phenothiazine (free state) is referred to the preparation method in international application No. WO2019205854A1.

Examples

Example 1: Method 1 of preparing the crystal Form A of 2-(1-(4-(4-methylpiperazin-1-yl)phenyl)ethyl)-10H-phenothiazine hydrochloride

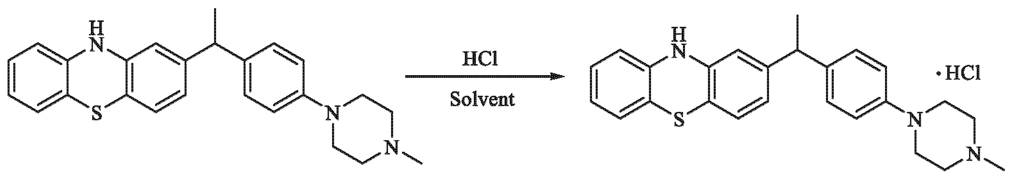


2-(1-(4-(4-methylpiperazin-1-yl)phenyl)ethyl)-10H-phenothiazine (free state)

2-(1-(4-(4-methylpiperazin-1-yl)phenyl)ethyl)-10H-phenothiazine hydrochloride

Nitrogen was introduced into a 50L reaction kettle to replace the gas therein, and then 1.70 kg of 2-(1-(4-(4-methylpiperazin-1-yl)phenyl)ethyl)-10R-phenothiazine (free state) and 17.78 kg of methanol were added to the reaction kettle under the nitrogen atmosphere and stirred. A mixed solution of 0.41 kg of concentrated hydrochloric acid (36% by mass) and 0.82 kg of methanol was added dropwise to the reaction kettle at 25±5°C. After approximately 1 h of dropwise addition was completed, the reaction mixture was stirred at a temperature kept at 25±5°C for 2-4 h for crystallization and then filtered. The filter cake was washed with 1.50 kg of methanol and dried under vacuum at a pressure of -0.08MPa to -0.10MPa at 40-50°C for 12-16 h. The resulting product was collected to obtain the crystal Form A of 2-(1-(4-(4-methylpiperazin-1-yl)phenyl)ethyl)-10R-phenothiazine hydrochloride in total of 1.64 kg with a yield of 88.3%, which was identified as crystal Form A by XRPD detection (XRPD pattern is shown in FIG. 1).

The resulting crystal Form A has a purity of 99.76%, and a maximum single impurity content of 0.068%. The total impurity and single impurity were less than the control limit of <0.15% as stipulated in the Technical Guidelines for the Study of Impurities in Chemical Drugs published by the State Pharmaceutical Administration (SDA) and the ICHQ3A (the maximum daily dosage of this product per day was ≤2 g/day). The profile of purity detection is shown in FIG. 4, wherein the major peak named as DA414 represents crystal Form A of 2-(1-(4-(4-methylpiperazin-1-yl)phenyl)ethyl)-10H-phenothiazine hydrochloride.

Data for nuclear magnetic resonance: $^{1}$H NMR (400 MHz, DMSO) δ=11.27 (s, 1H), 8.64 (s, 1H), 7.09 (d, J = 8.6 Hz, 2H), 6.92 (m, 4H), 6.80 (d, J = 7.9 Hz, 1H), 6.71 (t, J = 8.6 Hz, 2H), 6.64 (d, J = 7.9 Hz , 1H), 6.56 (d, J = 1.2 Hz, 1H), 3.90 (m, 1H), 3.60 (d, J = 73.7 Hz, 2H), 3.39 (s, 2H), 3.16 (d, J = 9.1 Hz, 4H), 2.77 (s, 3H), 1.45 (d, J = 7.2 Hz, 3H) (see FIG. 5 for $^{1}$H NMR spectrum).

Example 2: Method 2 of preparing the crystal Form A of 2-(1-(4-(4-methylpiperazin-1-yl)phenyl)ethyl)-10H-phenothiazine hydrochloride

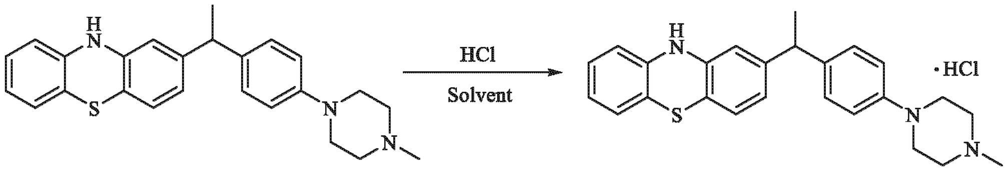


2-(1-(4-(4-methylpiperazin-1-yl)phenyl)ethyl)-10H-phenothiazine (free state)

2-(1-(4-(4-methylpiperazin-1-yl)phenyl)ethyl)-10H-phenothiazine hydrochloride

Nitrogen was introduced into a 50L reaction kettle to replace the gas therein, and then 1.50 kg of 2-(1-(4-(4-methylpiperazin-1-yl)phenyl)ethyl)-10H-phenothiazine (free state), 7.11 kg of acetone and 1.50 kg of water were added to the reaction kettle under the nitrogen atmosphere and stirred. A mixed solution of 0.41 kg concentrated hydrochloric acid (36% by mass), 0.68 kg of acetone and 0.15 kg of water was added dropwise to the reaction kettle at 25±5°C. After approximately 1 h of dropwise addition was completed, the reaction mixture was stirred for crystallization at a temperature kept at 25±5°C for 2-4 h and then filtered. The filter cake was washed with 1.50 kg of acetone and dried under vacuum at a pressure of -0.08MPa to -0.10MPa at 40-50°C for 12-16 h. The resulting product was collected to obtain 1.30 kg of crystal Form A of 2-(1-(4-(4-methylpiperazin-1-yl)phenyl)ethyl)-10R-phenothiazine hydrochloride with a yield of 79.5%, which was identified as crystal Form A by XRPD detection (XRPD pattern is shown in FIG. 2).

Example 3: Method of preparing the crystal Form B of 2-(1-(4-(4-methylpiperazin-1-yl)phenyl)ethyl)-10H-phenothiazine hydrochloride

Approximately 5 mg of crystal Form A of 2-(1-(4-(4-methylpiperazin-1-yl)phenyl)ethyl)-10H-phenothiazine hydrochloride was weighed, added with 0.2 mL of N,N-dimethylformamide, and dissolved by ultrasonication. The solution was filtered into a 5 mL vial with a stirrer, and then dropwise added with 4 mL of ethyl acetate under stirring at room temperature. A large amount of solid was precipitated. The mixture was filtered. The filter cake was dried under vacuum at room temperature overnight, which was identified as crystal Form B by XRPD detection (XRPD pattern of crystal Form B is shown in FIG. 3).

Example 4: XRPD detection of the crystal Form A of 2-(1-(4-(4-methylpiperazin-1-yl)phenyl)ethyl)-10H-phenothiazine hydrochloride

The detection was performed according to Chinese Pharmacopoeia (2020 edition) -Part Four -General Principles 0451-X-ray diffraction-Method 2- X-ray powder diffraction method.

Table 1 Parameters for XRPD detection of crystal Form A

| **X-Ray Diffractometer (for XRPD)** | |
|---|---|
| Model | D8 Advance |
| Number | CA312 |
| Technical specifications | Cu-Kα radiation with λ= 1.54 Å (40 kV, 40 mA), goniometer of θ-2θ, nickel filter, SSD160-2 detector |
| Acquisition software | DIFFRAC.MEA. CENTER |
| Internal standard | Corundum ($Al_2O_3$) |
| Analysis | MDI Jade 6 |
| software | |
| Scanning range | 3-40° 2θ |
| Step size | 0.02° 2θ |
| Speed | 0.1s/0.2s/step |
| Sample amount | >1 mg |
| Note | Unless otherwise stated, samples are not ground prior to detection. |

The XRPD pattern of the crystal form of 2-(1-(4-(4-methylpiperazin-1-yl)phenyl)ethyl)-10H-phenothiazine

hydrochloride is shown in FIG. 1. XRPD data is shown in Table 2. This crystal form was named as crystal Form A.

Table 2 XRPD data of crystal Form A

| Number | 2θ | Intensity | Number | 2θ | Intensity | Number | 2θ | Intensity | Number | 2θ | Intensity |
|---|---|---|---|---|---|---|---|---|---|---|---|
| **1** | 5.44 | 4.2% | **9** | 16.54 | 18.1% | **17** | 22.92 | 22.1% | **25** | 28.54 | 10.3% |
| **2** | 9.85 | 3.3% | **10** | 17.34 | 7.5% | **18** | 23.40 | 100.0% | **26** | 29.03 | 54.8% |
| **3** | 10.68 | 32.2% | **11** | 17.84 | 31.4% | **19** | 25.11 | 12.5% | **27** | 29.67 | 6.2% |
| **4** | 11.38 | 9.4% | **12** | 18.57 | 98.7% | **20** | 25.51 | 13.0% | **28** | 31.61 | 1.9% |
| **5** | 12.63 | 14.0% | **13** | 20.89 | 25.3% | **21** | 25.88 | 19.3% | **29** | 32.05 | 6.9% |
| **6** | 13.75 | 7.8% | **14** | 21.08 | 51.2% | **22** | 26.32 | 5.6% | **30** | 32.49 | 5.5% |
| **7** | 14.36 | 88.7% | **15** | 21.52 | 9.1% | **23** | 26.65 | 2.3% | **31** | 33.55 | 11.7% |
| **8** | 16.06 | 10.4% | **16** | 22.14 | 84.6% | **24** | 27.50 | 34.3% | **32** | 34.63 | 7.8% |

Example 5: XRPD detection of the crystal Form B of 2-(1-(4-(4-methylpiperazin-1-yl)phenyl)ethyl)-10H-phenothiazine hydrochloride.

The detection was performed according to Chinese Pharmacopoeia (2020 edition) -Part Four -General Principles 0451-X-ray diffraction-Method 2- X-ray powder diffraction method.

Table 3 Parameters for XRPD detection of crystal Form B

| **X-Ray Diffractometer (for XRPD)** | |
|---|---|
| Model | D8 Advance |
| Number | CA312 |
| Technical specifications | Cu-Kα radiation with λ= 1.54 Å (40 kV, 40 mA), goniometer of θ-2θ, nickel filter, SSD160-2 detector |
| Acquisition software | DIFFRAC.MEA.CENTER |
| Internal standard | Corundum ($Al_2O_3$) |
| Analysis software | MDI Jade 6 |
| Scanning range | 3-40° 2θ |
| Step size | 0.02° 2θ |
| Speed | 0.1s/0.2s/step |
| Sample amount | >1 mg |
| Note | Unless otherwise stated, samples are not ground prior to detection. |

The XRPD pattern of the crystal form of 2-(1-(4-(4-methylpiperazin-1-yl)phenyl)ethyl)-10H-phenothiazine hydrochloride is shown in FIG. 3. XRPD data is shown in Table 4. This crystal form was named as crystal Form B.

Table 4 XRPD data of crystal Form B

| **Number** | 2θ | Intensity | **Number** | 2θ | Intensity |
|---|---|---|---|---|---|
| **1** | 5.41 | 5.2% | **5** | 27.62 | 6.6% |
| **2** | 10.92 | 3.0% | **6** | 28.91 | 4.3% |
| **3** | 16.46 | 28.1% | **7** | 33.41 | 6.7% |
| **4** | 22.01 | 100% | | | |

Example 6: TGA detection of the crystal Form A

Table 5 Parameters for TGA detection

| | | |
|---|---|---|
| Instrument | Model | TGA2 |
| | Number | CA201 |
| | Control software | STARe software |
| | Analysis software | STARe software |
| | Sample tray | Alumina Crucible |
| Parameters | Sample amount | 1 mg-10 mg |
| | Protective gas | Nitrogen |
| | Gas flow rate | 50 mL/min |
| | Commonly used detection method | Segment 1<br>Start temp 30.0°C<br>End temp 350.0°C<br>Heating rate 10.0 k/min |

The TGA profile of crystal Form A is shown in FIG. 6.
The results of TGA detection showed that the sample of crystal Form A has a weight loss of about 0.1586% when warmed up to 120°C, indicating that the crystal Form A is anhydrous.

Example 7: DSC detection of crystal Form A

Table 6 Parameters for DSC detection

| | | |
|---|---|---|
| Instrument | Model | DSC3 |
| | Number | CA200 |
| | Control software | STARe software |
| | Analysis software | STARe software |
| | Sample tray | Aluminium crucible (with perforated lid) |
| Parameters | Sample amount | 0.5 mg-5 mg |
| | Protective gas | Nitrogen |
| | Gas flow rate | 50 mL/min |
| | Commonly used detection method | Segment 1<br>Start temp 30.0°C<br>End temp 300.0°C<br>Heating rate 10.0 k/min |

The DSC pattern of crystal Form A is shown in FIG. 7.
The DSC pattern showed that the crystal Form A has a single heat absorption peak and a melting point of about 265.31°C, indicating that the crystal Form A has good thermodynamic stability.

Example 8: Ion chromatography (IC) of crystal Form A

Table 7 Parameters for ion chromatography (IC)

| Ion chromatography (IC) | | |
|---|---|---|
| Instrument | Model | ICS-5000 |
| | Workstation/Analysis Software | Chromeleon Console |
| Parameters in Cation Detection System Method | Column | Dionex IonPac™ AG18 RFIC™, 4*50 mm; Dionex IonPac™ AS 18 RFIC™, 4*250 mm |
| | Column temperature | 30°C |
| | Injector temperature | Room temperature |
| | Solvent in sample | Ultrapure water |
| | Eluent solution | 30 mM potassium hydroxide aqueous solution |
| | Flow rate | 1.0 mL/min |
| | Collection time | 20 min |
| | Suppressor current | 75 mA |
| | Injection volume | 25 μL |

The results of ion chromatography (IC) show a salt formation ratio of 1:1, indicating that 2-(1-(4-(4-methylpiperazin-1-yl)phenyl)ethyl)-10R-phenothiazine binds to HCl in a ratio of 1:1.

Example 9: Other methods of preparing crystal Form A

(1) Volatilization for crystallization

Experimental procedure: Approximately 10 mg of crystal Form A of 2-(1-(4-(4-methylpiperazin-1-yl)phenyl)ethyl)-10H-phenothiazine hydrochloride was weighed into a 5 mL vial, and dissolved with a certain volume of the mixed solvent listed in Table 8. The mixture was filtered through a 0.45 μm PTFE filter into a new 5 mL vial which was left open for volatilization at different temperatures. The resulting solid was collected and subjected to XRPD detection. The results are shown in Table 8, indicating that the crystal Form A product was obtained.

Table 8 Summary of volatilization experiment results

| Number | Solvent/solvent, v:v | Total volume of solvent (mL) | Temperature (°C) | Crystal form as a solid |
|---|---|---|---|---|
| 1 | Water/ethanol, 1:3 | 1 | 40 | Crystal Form A of hydrochloride salt |
| 2 | Water/methanol, 1:3 | 1 | 40 | Crystal Form A of hydrochloride salt |
| 3 | Water/trifluoroethanol, 1:3 | 0.4 | 40 | Crystal Form A of hydrochloride salt |
| 4 | Water/isopropanol, 1:2 | 0.4 | 40 | Crystal Form A of hydrochloride salt |
| 5 | Acetone/trifluoroethano 1, 1:1 | 1 | 40 | Crystal Form A of hydrochloride salt |
| 6 | Isopropanol/trifluoroeth anol, 1:1 | 1 | 40 | Crystal Form A of hydrochloride salt |
| 7 | Acetonitrile/trifluoroeth anol, 1:1 | 1 | 40 | Crystal Form A of hydrochloride salt |
| 8 | Toluene/trifluoroethanol , 2:1 | 0.2 | 60 | Crystal Form A of hydrochloride salt |

(2) Anti-solvent addition method

Approximately 5 mg of crystal Form A of 2-(1-(4-(4-methylpiperazin-1-yl)phenyl)ethyl)-10H-phenothiazine hydrochloride was weighed into a 5 mL vial, and then dissolved with the first solvent in Table 9. The mixture was filtered

through a 0.45 μm PTFE filter into a new 5 mL vial. To the resulting clear solution, the second solvent in Table 9 was added dropwise under stirring until a solid was precipitated. If no solids were precipitated after a total volume of 5 mL of solvent was added, the sample was transferred to 5°C and stirred magnetically. After stirring overnight, the samples that were still clear were then transferred to room temperature, and evaporated or spin-dried. The solid was collected by centrifugation and subjected to XRPD detection. The results are shown in Table 9 and the crystal Form A of the hydrochloride salt was obtained.

Table 9 Summary of experimental results of anti-solvent addition

| Number | First solvent/second solvent, v:v | Total volume of solvent (mL) | Crystal form as a solid |
|---|---|---|---|
| 9 | Methanol/acetone, 1:5 | 5 | Crystal Form A of hydrochloride salt |
| 10 | Ethanol/methyl tert-butyl ether, 1:5 | 5 | Crystal Form A of hydrochloride salt |
| 11 | Trifluoroethanol/1,4-dioxane, 1:25 | 5.2 | Crystal Form A of hydrochloride salt |
| 12 | Dimethylsulfoxide/toluene, 1:20 | 2.1 | Crystal Form A of hydrochloride salt |
| 13 | N,N-Dimethylformamide/ethyl acetate, 1:20 | 4.2 | Crystal Form A of hydrochloride salt |
| 14 | N-methylpyrrolidone/acetone, 1:40 | 4.1 | Crystal Form A of hydrochloride salt |

(3) Reverse anti-solvent addition method

Approximately 5 mg of crystal Form A of 2-(1-(4-(4-methylpiperazin-1-yl)phenyl)ethyl)-10H-phenothiazine hydrochloride was weighed into a 5 mL vial, and completely dissolved with the first solvent in Table 10. The mixture was filtered through a 0.45 μm PTFE filter into a new 5 mL vial. The resulting clear solution was added dropwise to 3 mL of the second solvent in Table 10 under stirring. If no solid was precipitated, the sample was magnetically stirred at 5°C. After stirring overnight, the sample which was still clear was transferred to room temperature to be evaporated or spin-dried. The solid was collected by centrifugation and subjected to XRPD detection. The results are shown in Table 10, indicating that only the crystal Form A was obtained.

Table 10 Summary of experimental results of reverse anti-solvent addition

| Number | First solvent | Second solvent | First solvent volume (mL) | Crystal form as a solid |
|---|---|---|---|---|
| 15 | Methanol | Acetone | 1 | N/A |
| 16 | Ethanol | Methyl tert-butyl ether | 1 | N/A |
| 17 | Trifluoroethanol | 1,4-dioxane | 0.2 | N/A |
| 18 | Dimethyl Sulfoxide | Toluene | 0.1 | Crystal Form A of hydrochloride salt |
| 19 | N,N-dimethylformamide | Ethyl acetate | 0.2 | Crystal Form A of hydrochloride salt |
| 20 | N-methylpyrrolidone | Acetone | 0.1 | Crystal Form A of |
| | | | | hydrochloride salt |
| Note: N/A means that not enough samples were obtained for detection. | | | | |

(4) Cooling crystallization method

Approximately 10 mg of the crystal Form A of 2-(1-(4-(4-methylpiperazin-1-yl)phenyl)ethyl)-10H-phenothiazine hydrochloride was weighed into a 5 mL vial, and added with a certain volume of a single solvent or mixed solvent in Table 11. This mixture was completely dissolved by ultrasonication at 50°C, and filtered with a 0.45 μm PTFE filter into a new 5 mL vial. The obtained clear filtrate was placed in a biochemical incubator at 50°C for 120 min and then cooled down to 5°C at a rate of 0. 1°C/min under stirring throughout the cooling process. If a solid was precipitated, it was collected by centrifugation and subjected to the XRPD detection. The results are shown in Table 11.

Table 11 Summary of experimental results of cooling crystallization

| Number | Slovent/ Slovent, v:v | Total volume of solvent (mL) | Crystal form as solid |
|---|---|---|---|
| 21 | Methanol | 1 | Crystal Form A of hydrochloride salt |
| 22 | Ethanol | 2.5 | Crystal Form A of hydrochloride salt |
| 23 | Methanol/tetrahydrofuran, 1:3 | 1.2 | Crystal Form A of hydrochloride salt |
| 24 | Ethanol/acetonitrile, 2:1 | 0.7 | Crystal Form A of hydrochloride salt |
| 25 | Trifluoroethanol/isopropyl acetate, 1:4 | 5 | Oil (no solid) |
| 26 | N,N-dimethylformamide/isopropanol, 1:5 | 3.6 | Crystal Form A of hydrochloride salt |
| 27 | Dimethylsulfoxide/2-methyltetrahydrofuran, 1:4 | 2.4 | Solution (no solid) |
| 28 | N-methylpyrrolidone/toluene, 1:4 | 3 | Solution (no solid) |

(5) Gas-liquid diffusion method

Approximately 10 mg of the crystal Form A of 2-(1-(4-(4-methylpiperazin-1-yl)phenyl)ethyl)-10H-phenothiazine hydrochloride was weighed into a 5 mL vial, and dissolved with a certain volume of the first solvent (single solvent or mixed solvent) in Table 12. The mixture was filtered with a 0.45 μm PTFE filter into a new 5 mL vial. A 20 mL vial was added with about 3 mL of the second solvent. The 5 mL vial containing the clear solution without lid was placed in 20 mL vial. To the 20 mL vial, about 3 mL of the second solvent was added. Then the 20ml vial was sealed and placed at room temperature for about one week. The solid was collected and subjected to XRPD detection. The results are shown in Table 12.

Table 12 Summary of experimental results of gas-liquid diffusion

| Number of sample | First solvent, v:v | Second solvent | Crystal form as solid |
|---|---|---|---|
| 29 | Methanol | Methyl tert-butyl ether | Crystal Form A of hydrochloride salt |
| 30 | Ethanol | n-Hexane | Crystal Form A of hydrochloride salt |
| 31 | Trifluoroethanol | Dichloromethane | Crystal Form A of hydrochloride salt |
| 32 | Dimethyl sulfoxide | Ethyl acetate | Solution (no solid) |
| 33 | N-methylpyrrolidone | Acetone | Solution (no solid) |
| 34 | n-Heptane/ethanol, 1:2 | Acetone | Crystal Form A of hydrochloride salt |
| 35 | 1,4-Dioxane/Dimethylsulfoxide, 4:1 | Methyl tert-butyl ether | Solution (no solid) |
| 36 | Isopropyl acetate/methanol, 1:2 | Dichloromethane | Crystal Form A of hydrochloride salt |
| 37 | Isopropanol /N-methyl pyrrolidone, 4:1 | Ethyl acetate | Solution (no solid) |

(6) Polymer induction method

Approximately 10 mg of the crystal Form A of 2-(1-(4-(4-methylpiperazin-1-yl)phenyl)ethyl)-10H-phenothiazine hydrochloride was weighed into a 5 mL vial, dissolved with a certain volume of a mixed solvent in Table 13, and filtered with a 0.45 μm PTFE filter, and 1-2 mg of polymer was added to the filtrate. The vial was sealed with a sealing film with holes, and placed in the room temperature for slow volatilization. The results are shown in Table 13, indicating that the crystal Form A was obtained.

Table 13 Summary of experimental results of polymer induction

| Number | Solvent/ Solvent, v:v | Polymer | Crystal form as solid |
|---|---|---|---|
| 38 | Water/ethanol, 1:1 | Copovidone | Crystal Form A of hydrochloride salt |

(continued)

| Number | Solvent/ Solvent, v:v | Polymer | Crystal form as solid |
|---|---|---|---|
| 39 | Water/acetonitrile, 1:1 | Polyethyleneglycol | Oil (no solid) |
| 40 | Water/acetone, 1:1 | Ethylcellulose | Crystal Form A of hydrochloride salt |
| 41 | Water/trifluoroethanol, 1:1 | Polyvinylpyrrolidone | N/A |
| 42 | Ethanol/dichloromethane, | Crospovidone | N/A |
| Note: N/A represents that not enough samples were obtained for the detection. | | | |

The XRPD detection results show that the crystal forms of the samples prepared in Example 9 were all crystal Form A (XRPD pattern is not shown).

Example 10: Other methods of preparing crystal Form B

(1) Anti-solvent addition method

Approximately 5 mg of the crystal Form A of 2-(1-(4-(4-methylpiperazin-1-yl)phenyl)ethyl)-10H-phenothiazine hydrochloride was weighed into a 5 mL vial, and completely dissolved with the first solvent in Table 14. The solution was filtered with a 0.45 μm PTFE filter into a new 5 mL vial. The second solvent in Table 14 was added dropwise to the obtained clear solution under stirring until a solid was precipitated. The solid was collected by centrifugation and subjected to XRPD detection. The results are shown in Table 14, indicating that the crystal Form B was obtained.

Table 14 Summary of experimental results of anti-solvent addition

| Number | First solvent/Second solvent, v:v | Total volume of solvent (mL) | Crystal form as solid |
|---|---|---|---|
| 43 | N,N-dimethylformamide/ethyl acetate, 1:20 | 4.2 | Crystal Form B of hydrochloride salt |

(2) Polymer induction method

Approximately 10 mg of the crystal Form A of 2-(1-(4-(4-methylpiperazin-1-yl)phenyl)ethyl)-10H-phenothiazine hydrochloride was weighed into a 5 mL vial, and dissolved with a certain volume of a mixed solvent in Table 15. The solution was filtered with a 0.45 μm PTFE filter. 1-2 mg of a polymer was added to the filtrate. The vial was sealed with a sealing film with holes and placed in the room temperature for slow volatilization. The results are shown in Table 15, indicating that the crystal Form B was obtained.

Table 15 Summary of experimental results of polymer induction

| Number | Solvent/solvent, v:v | Polymer | Crystal form as solid |
|---|---|---|---|
| 44 | Water/methanol, 1:1 | Hydroxypropylmethylcellulose | Crystal Form B of hydrochloride salt |

The XRPD detection results show that the crystal form of the samples prepared in example 10 were all crystal Form B (XRPD pattern was not shown).

Example 11: Study on hygroscopicity

Hygroscopicity was assessed by dynamic vapor sorption (DVS). The results show that the crystal Form A was slightly hygroscopic with a vapor sorption of about 0.36% at 25°C/80% RH, and the crystal form remained unchanged before and after the vapor sorption experiment.

The results of the study on hygroscopicity show that the crystal Form A has lower hygroscopicity than the crystal Form B, indicating a better druggability.

Example 12: Comparative study on solubility

According to the method of solubility measurement in the General Notices of Chinese Pharmacopoeia (2020

edition), 2-(1-(4-(4-methylpiperazin-1-yl)phenyl)ethyl)-10H-phenothiazine (free state), the crystal Form A of 2-(1-(4-(4-methylpiperazin-1-yl)phenyl)ethyl)-10H-phenothiazine hydrochloride, and the crystal Form B of 2-(1-(4-(4-methylpiperazin-1-yl)phenyl)ethyl)-10H-phenothiazine hydrochloride, were added to a certain volume of solvent at 25°C±2°C respectively, and shaken strongly for 30 seconds every 5 minutes. The dissolution phenomena was observed within 30 minutes, and the solubility was measured.

Table 16 Data for solubility

| Compound | Solubility | |
|---|---|---|
| | Water | Hydrochloric acid solution of pH 1.0 |
| 2-(1-(4-(4-methylpiperazin-1-yl)phenyl)ethyl)-10H-phenothiazine (free state) | <10ng/mL | 0.034mg/mL |
| Crystal Form A of 2-(1-(4-(4-methylpiperazin-1-yl)phenyl)ethyl)-10H-phenothiazine hydrochloride | 0.11 mg/mL | 0.5mg/mL |
| Crystal Form B of 2-(1-(4-(4-methylpiperazin-1-yl)phenyl)ethyl)-10H-phenothiazine hydrochloride | <0.01mg/mL | <0.05mg/mL |
| 2-(1-(4-(4-methylpiperazin-1-yl)phenyl)ethyl)-10H-phenothiazine hydrochloride | 0.077mg/mL | 0.35mg/mL |

wherein, 2-(1-(4-(4-methylpiperazin-1-yl)phenyl)ethyl)-10H-phenothiazine hydrochloride was obtained by method 1 in Example 17.

The results show that in the aqueous system, the solubility of the crystal Form A of 2-(1-(4-(4-methylpiperazin-1-yl)phenyl)ethyl)-10H-phenothiazine hydrochloride was absolutely superior to that of 2-(1-(4-(4-methylpiperazin-1-yl)phenyl)ethyl)-10R-phenothiazine (free state), 2-(1-(4-(4-methylpiperazin-1-yl)phenyl)ethyl)-10H-phenothiazine hydrochloride, and the crystal Form B of 2-(1-(4-(4-methylpiperazin-1-yl)phenyl)ethyl)-10R-phenothiazine hydrochloride. In a hydrochloric acid solution system of pH 1.0 (roughly the pH value of human gastric fluid), the solubility of the crystal Form A of 2-(1-(4-(4-methylpiperazin-1-yl)phenyl)ethyl)-10H-phenothiazine hydrochloride was absolutely superior to that of 2-(1-(4-(4-methylpiperazin-1-yl)phenyl)ethyl)-10R-phenothiazine (free state), 2-(1-(4-(4-methylpiperazin-1-yl)phenyl)ethyl)-10H-phenothiazine hydrochloride, and the crystal Form B of 2-(1-(4-(4-methylpiperazin-1-yl)phenyl)ethyl)-10H-phenothiazine hydrochloride, indicating that the crystal Form A had better physical and chemical properties, and better druggability. Surprisingly, the solubility in water of the crystal Form B of 2-(1-(4-(4-methylpiperazin-1-yl)phenyl)ethyl)-10H-phenothiazine hydrochloride was higher than that of free 2-(1-(4-(4-methylpiperazin-1-yl)phenyl)ethyl)-10R-phenothiazine by 1000 times.

In addition, it was found that the crystal forms obtained by crystallization from other salts of 2-(1-(4-(4-methylpiperazin-1-yl)phenyl)ethyl)-10H-phenothiazine, such as citrate, fumarate, and maleate salts, had essentially the same solubility in water and hydrochloric acid solution at pH 1.0 compared to the corresponding salts, and there was no significant improvement.

Example 13: Stability assay of crystal form

(1): Stability assay of crystal Form A

Experiment 1: Stability assay for temperature and humidity

Assay 1: The samples of crystal Form A were placed under long-term condition (25°C/60% RH) and accelerated condition (40°C/75% RH) for one week, and the change on crystal form was detected. The results show that after the samples of crystal Form A were placed under the two test conditions for one week, its purity was basically unchanged, the chemical stability was good, and no changes in crystal form were observed. The comparative XRPD patterns of the sample before and after stability assay are shown in FIG. 8.

Assay 2: Approximately 10 mg of the crystal Form A of 2-(1-(4-(4-methylpiperazin-1-yl)phenyl)ethyl)-10H-phenothiazine hydrochloride was weighed into a 5 mL vial, and the 5 mL vial was left open and placed in a corresponding container with a constant humidity. The container was sealed and placed under room temperature. After 1 week, the solid was collected and subjected to XRPD detection. No changes in crystal form were observed. The results are shown in Table 17.

Table 17 Results of stability assay for temperature and humidity

| Temperature-humidity (°C-RH%) | Crystal form as solid |
|---|---|
| RT-75%RH | Crystal Form A of hydrochloride salt |
| RT-97%RH | Crystal Form A of hydrochloride salt |

Experiment 2: Mechanical stability assay

Approximately 10 mg of the crystal Form A of 2-(1-(4-(4-methylpiperazin-1-yl)phenyl)ethyl)-10H-phenothiazine hydrochloride was weighed into a mortar, ground with 0.05 ml of wetting agent for 5 min and repeated three times. The solid was collected and subjected to XRPD detection, which shows that the crystal form of the sample was unchanged. The results are shown in Table 18.

Table 18 Results of mechanical stability assay

| Wetting agent | Wetting agent volume (mL) | Crystal form as solid |
|---|---|---|
| Water | 0.15 | Crystal Form A of hydrochloride salt |
| Ethanol | 0.15 | Crystal Form A of hydrochloride salt |
| Ethyl acetate/isopropanol, 1:1 | 0.15 | Crystal Form A of hydrochloride salt |

Experiment 3: Other stability assays

Assay 1: Approximately 10 mg of the crystal Form A of 2-(1-(4-(4-methylpiperazin-1-yl)phenyl)ethyl)-10H-phenothiazine hydrochloride was weighed into a 5 mL vial, and added with 1 mL of a single solvent or mixed solvent listed in Table 19. The resulting suspension was magnetically stirred at 5°C for approximately 3 days. The solid was collected by centrifugation and subjected to XRPD detection. The sample did not undergo crystal transformation. The results are shown in Table 19.

Table 19 Result of slurry conversion experiment at low temperature

| Solvent/ Solvent, v:v | Crystal form as solid |
|---|---|
| Ethanol | Crystal Form A of hydrochloride salt |
| Isopropanol/dichloromethane, 1:2 | Crystal Form A of hydrochloride salt |
| Methyl isobutyl ketone/acetonitrile, 1:1 | Crystal Form A of hydrochloride salt |
| Isopropyl acetate/tetrahydrofuran, 1:1 | Crystal Form A of hydrochloride salt |
| Toluene/acetone, 1:2 | Crystal Form A of hydrochloride salt |
| Ethyl acetate/trifluoroethanol, 4:1 | Crystal Form A of hydrochloride salt |
| Methyl tert-butyl ether/2-methyltetrahydrofuran, 2:1 | Crystal Form A of hydrochloride salt |

Assay 2: Approximately 10 mg of the crystal Form A of 2-(1-(4-(4-methylpiperazin-1-yl)phenyl)ethyl)-10H-phenothiazine hydrochloride was weighed into a 5 mL vial, and added with 0.5 mL of a single solvent or mixed solvent listed in Table 20 to obtain a suspension. The suspension was magnetically stirred at room temperature for approximately 3 days. Afterwards, the solid was collected by centrifugation and subjected to XRPD detection. The sample did not undergo crystal transformation. The results are shown in Table 20.

Table 20 Result of slurry conversion experiment at room temperature

| Solvent/ Solvent, v:v | Crystal form as solid |
|---|---|
| Methyl isobutyl ketone/acetonitrile, 1:4 | Crystal Form A of hydrochloride salt |
| Methyl tert-butyl ether/dichloromethane, 1:1 | Crystal Form A of hydrochloride salt |
| Isopropanol/1,4-dioxane, 1:1 | Crystal Form A of hydrochloride salt |

Assay 3: Approximately 10 mg of the crystal Form A of 2-(1-(4-(4-methylpiperazin-1-yl)phenyl)ethyl)-10H-phenothiazine hydrochloride was weighed into a 5 mL vial, and added with 0.5 mL of a single solvent or mixed solvent listed in Table 21. The resulting suspension was magnetically stirred at 50°C for approximately 3 days. Afterwards, the solid was collected by centrifugation and subjected to XRPD detection. The sample did not undergo crystal transformation. The results are shown in Table 21.

Table 21 Result of slurry conversion experiment at high temperature

| Solvent/ Solvent, v:v | Crystal form as solid |
|---|---|
| Isopropanol | Crystal Form A of hydrochloride salt |
| n-Heptane/Ethanol, 1:1 | Crystal Form A of hydrochloride salt |
| Tetrahydrofuran/ethyl acetate, 1:1 | Crystal Form A of hydrochloride salt |
| Methyl isobutyl ketone/ethanol, 1:2 | Crystal Form A of hydrochloride salt |
| 1,4-Dioxane/methyl tert-butyl ether, 1:4 | Crystal Form A of hydrochloride salt |
| Toluene/n-heptane, 1:1 | Crystal Form A of hydrochloride salt |

(2) Stability assay of crystal Form B

Assay: A certain amount of crystal Form B sample was weighed into a sample vial. Then the sample vial was sealed and left at room temperature for 2 days and 5 days. Then, a certain amount of samples in the sample vial was taken and subjected to XRPD detection. The results show that the crystal Form B sample, after being sealed and left at room temperature for 2 days, began to undergo crystal transformation into crystal Form A; and after being sealed and left at room temperature for 5 days, the crystal Form B sample partially transformed into crystal Form A, indicating that the crystal Form B was unstable. The XRPD pattern of the crystal Form B in stability assay is shown in FIG. 9, which indicates that the crystal Form A has a better stability than that of the crystal Form B.

In addition, the crystal Form A has good properties such as compressibility, flowability, specific surface area, stacking density, porosity, and particle size, making it very suitable for the development and preparation as pharmaceutical formulations. However, the crystal Form B does not have these properties.

In addition, the crystal Form A and the crystal Form B were subjected to influencing factor test. After being placed under high temperature (60°C), high humidity (25°C/90%$\pm$5% RH), and illumination (45001x$\pm$5001x) conditions for 30 days, the crystal Form A showed a better stability and a lower impurity content than that of the crystal form B.

Moreover, the crystal Form A had a better stability and a lower total impurity content compared to the corresponding free compound.

(3) Comparative stability assay of crystal Form A and crystal Form B

According to the Chinese Pharmacopoeia 2020 and ICHQ1A stability study guidelines, the crystal Form A and crystal Form B were placed under accelerated (40°C $\pm$ 2°C, 75% RH $\pm$ 10% RH) conditions for 6 months, and the total impurity content of the crystal Form A and crystal Form B was detected. The results are shown in Table 22 (stability assay of different crystal forms), showing that after being placed under accelerated conditions for 6 months, the crystal Form A exhibited an increase from 0.25% to 0.41% in total impurity content, while the crystal Form B exhibited an increase from 0.28% to 0.88% in total impurity content, indicating that the stability of the crystal Form B is very poor, and the stability of the crystal Form A is better than that of the crystal Form B.

Table 22 Stability assay of different crystal forms

| Item | Comparative stability assay of crystal form | |
|---|---|---|
| Collection time point | On day 0, and at month 6 | |
| Detection items | Oxidized impurity, maximum unknown single impurity and total impurity | |
| Experimental condition | Accelerated condition (40°C $\pm$ 2°C, 75% RH $\pm$ 10% RH) | |
| Crystal Form | Crystal Form A | Crystal Form B |

(continued)

| Experimental result | On day 0 | At month 6 | On day 0 | At month 6 |
|---|---|---|---|---|
| | Total impurity: 0.25% | Total impurity: 0.41% | Total impurity: 0.28% | Total impurity: 0.88% |

Example 14: Comparative pharmacokinetic study

Experimental method: 3 healthy male SD rats were fasted overnight, and intragastrically administrated with 10 mg/ kg of 2-(1-(4-(4-methylpiperazin-1-yl)phenyl)ethyl)-10R-phenothiazine (free state) or 10 ml/ kg of the crystal Form A of 2-(1-(4-(4-methylpiperazin-1-yl)phenyl)ethyl)-10H-phenothiazine hydrochloride in suspension (0.5% CMC-Na as solvent). Before the administration and at 0.25, 0.5, 1, 2, 4, 8, 12, and 24 h after the administration, 200 μL of the whole blood was collected from the orbital venous plexus of rats, placed in a centrifugation tube containing EDTA-K2 anticoagulant, and then centrifuged at 3,000-4,000 rpm for 10 min. All the upper layer of plasma was transferred to another clean centrifugation tube. The concentration of 2-(1-(4-(4-methylpiperazin-1-yl)phenyl)ethyl)-10H-phenothiazine in the plasma sample was determined by LC-MS/MS, and the pharmacokinetic parameters were calculated using a non-atrial model.

Table 23 Pharmacokinetic parameters of rats intragastrically administered with drugs

| Compound | 2-(1-(4-(4-methylpiperazin-1-yl) phenyl) ethyl)-10H-phenothiazine (free state) | Crystal Form A of 2-(1-(4-(4-methylpiperazin-1-yl) phenyl) ethyl)-10H-phenothiazine hydrochloride |
|---|---|---|
| Administration route | Oral gavage | Oral gavage |
| Administration dose (mg/kg) | 10 | 10 |
| AUC0-t (h*ng/ml) | 1555.66±187.11 | 1817.50±142.48 |
| AUC0-inf (h*ng/ml) | 1902.96±403.61 | 2328.87±156.41 |
| Maximum plasma concentration Cmax (ng/ml) | 110.52±10.81 | 130.00±7.00 |
| Tmax (h) | 2.67±1.15 | 4.00±0.00 |
| Half life time $T_{1/2}$(h) | 9.28±2.42 | 10.44±1.24 |
| Bioavailability F% | 31.29±6.64 | 46.28±3.11 |
| Note: t represents 24 h in the table. | | |

The results show that, after oral administration, the crystal Form A of 2-(1-(4-(4-methylpiperazin-1-yl)phenyl) ethyl)-10H-phenothiazine hydrochloride was well absorbed in the rats, with a better plasma exposure and bioavailability compared to 2-(1-(4-(4-methylpiperazin-1-yl)phenyl)ethyl)-10R-phenothiazine (free state), indicating that the druggability of the crystal Form A was significantly improved compared to the free state of the compound.

Example 15: Study on the inhibition of ferroptosis by the compound of the present disclosure

Experimental method: In this example, a screening model for ferroptosis was constructed to investigate a ferroptosis inhibitor. Specifically, the screening model for ferroptosis was mainly based on the CCK8 cell viability test. Firstly, mouse hippocampal neuronal cells HT22 and human neuroblastoma SH-SY5Y cells were cultured in a dish. 80 μL of cells (5000 cells/well) in logarithmic growth phase were seeded to a 96-well plate per well, and then cultured in an incubator at 37°C, 5% $CO_2$ to allow adhesion of cells. After 24 h, 10 μL of the compound of the present disclosure at various concentrations prepared with the specified medium and 10 μL of Erastin (ferroptosis inducer) at a final concentration of 10 μM were added. 3 replicate wells were set for each compound to ensure the accuracy of the results. A positive control group (10 μL of a certain concentration of the compound Ferrostain-1 and 10 μL of Erastin (ferroptosis inducer) prepared with the same medium were added with reference to the above description). A blank control group (an equal volume of the specified medium and an equal volume of DMSO comprising no cells were added) and a solvent control group (an equal volume of the specified medium and an equal volume of DMSO comprising cells were added) were set up. Each group was also set up with 3 replicate wells to ensure the accuracy of the results. After the addition of the drug, the cells were incubated in an incubator for 24 h. 10 μL of CCK8 solution was added to each well, and the cells were further incubated in the incubator for 2-4 h. The absorbance at 450 nm was detected by a microplate reader to calculate the inhibition rate of ferroptosis by the drug. The following formula was used to calculate the inhibition rate:

$$\% \text{ Inhibition rate (IR)} = [1 - (A_{\text{experimental group}} - A_{\text{blank}}) / (A_{\text{solvent}} - A_{\text{blank}})] * 100\%$$

The inhibition rate change curve was fitted and the $IC_{50}$ was calculated by the software GraphPadPrism6.

Experimental results:

Table 24 $IC_{50}$ values of the compound of the present disclosure

| Cell | Ferrostain-1 $IC_{50}$ (nM) | $IC_{50}$ of crystal Form A of 2-(1-(4-(4-methylpiperazin-1-yl)phenyl)ethyl)-10H-phenothiazine hydrochloride (nM) |
|---|---|---|
| HT22 | 96.155 | 2.87 |
| SH-SY5Y | 53.935 | 1.409 |

As can be seen from the experimental results shown in Table 24, the crystal Form A of compound of 2-(1-(4-(4-methylpiperazin-1-yl)phenyl)ethyl)-10H-phenothiazine hydrochloride had an inhibitory effect on ferroptosis in several cell lines, and the inhibitory activity was significantly better than that of the positive control Ferrostain-1.

Example 16: Preparation of 2-(1-(4-(4-methylpiperazin-1-yl)phenyl)ethyl)-10H-phenothiazine

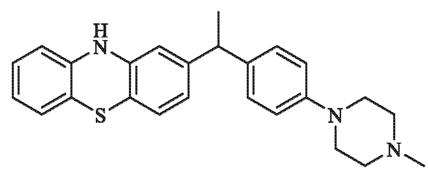

2-(1-(4-(4-methylpiperazin-1-yl)phenyl)ethyl)-10H-phenothiazine

The specific synthesis of 2-(1-(4-(4-methylpiperazin-1-yl)phenyl)ethyl)-10H-phenothiazine was referred to the preparation method in International Application No. WO2019205854A1. The product impurities were determined and the total impurity content was 1.36%.

Example 17: Preparation of 2-(1-(4-(4-methylpiperazin-1-yl)phenyl)ethyl)-10H-phenothiazine hydrochloride

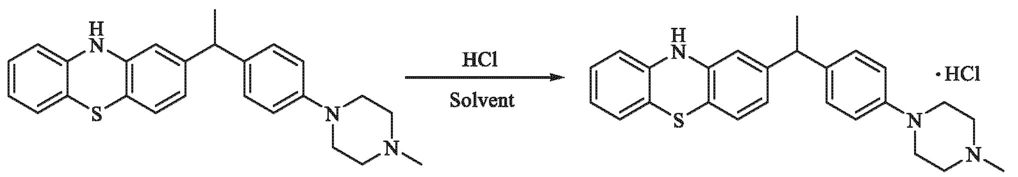


2-(1-(4-(4-methylpiperazin-1-yl)phenyl)ethyl)-10H-phenothiazine (free state)

2-(1-(4-(4-methylpiperazin-1-yl)phenyl)ethyl)-10H-phenothiazine hydrochloride

Method 1

5.0 g of 2-(1-(4-(4-methylpiperazin-1-yl)phenyl)ethyl)-10H-phenothiazine (12.45 mmol, 1.0 eq) was dissolved in 50 mL of acetone at 20-25°C. 1.14 mL of concentrated hydrochloric acid (13.70 mmol, 1.1eq) with a mass fraction of 36% was diluted to 4.5 mL with acetone, and then slowly added dropwise into the reaction solution. The mixed solution was stirred for 1 h, and then filtered. The filter cake was dried to obtain 4.4 g of 2-(1-(4-(4-methylpiperazin-1-yl)phenyl)ethyl)-10H-phenothiazine hydrochloride with a yield of 80.7%. $^1$H NMR (400 MHz, DMSO-d6) $\delta$=11.27 (s, 1H), 8.65 (s, 1H), 7.10 (d, J = 8.6 Hz, 2H), 6.93 (m, 4H), 6.81 (d, J = 7.9 Hz, 1H), 6.72 (t, J = 8.6 Hz, 2H), 6.65 (d, J = 7.9 Hz, 1H), 6.56 (d, J = 1.2 Hz, 1H), 3.91 (m, 1H), 3.70 (s, 2H), 3.35 (s, 4H), 3.17 (d, J = 9.1 Hz, 2H), 2.77 (s, 3H), 1.46 (d, J = 7.2 Hz, 3H), (See FIG. 10 for $^1$H NMR spectrum); $^{13}$C NMR (101 MHz, DMSO-d6) $\delta$=148.29, 146.87, 142.56, 138.00, 128.37, 127.87, 126.54,

122.09, 121.26, 116.89, 116.57, 114.91, 113.98, 52.47, 46.11, 43.12, 42.35, 21.91, (See FIG. 11 for $^{13}C$ NMR spectrum). It was confirmed though structure analysis that 2-(1-(4-(4-methylpiperazin-1-yl)phenyl)ethyl)-10H-phenothiazine was combined with hydrochloric acid at 1:1 in the resulting hydrochloride salt.

Method 2

The operating procedure was the same as method 1 except that the solvent acetone therein was replaced by ethanol to obtain the product of 2-(1-(4-(4-methylpiperazin-1-yl)phenyl)ethyl)-10H-phenothiazine hydrochloride, which was structurally confirmed to be the same as the product obtained by method 1.

Method 3

The operating procedure was the same as method 1 except that the solvent acetone therein was replaced by isopropanol to obtain the product of 2-(1-(4-(4-methylpiperazin-1-yl)phenyl)ethyl)-10H-phenothiazine hydrochloride, which was structurally confirmed to be the same as the product obtained by method 1.

Method 4

The operating procedure was the same as method 1 except that the solvent acetone therein was replaced by tetrahydrofuran to obtain the product of 2-(1-(4-(4-methylpiperazin-1-yl)phenyl)ethyl)-10H-phenothiazine hydrochloride, which was structurally confirmed to be the same as the product obtained by method 1.

The impurities of the above hydrochloride salt products were detected and the total impurity content was 0.10% to 0.25%.

Example 18: Preparation of 2-(1-(4-(4-methylpiperazin-1-yl)phenyl)ethyl)-10H-phenothiazine mesylate

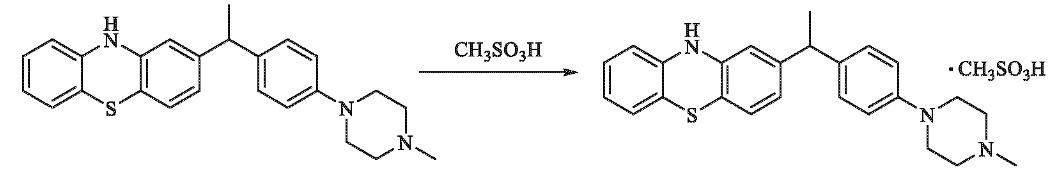


2-(1-(4-(4-methylpiperazin-1-yl)phenyl)ethyl)-10H-phenothiazine

2-(1-(4-(4-methylpiperazin-1-yl)phenyl)ethyl)-10H-phenothiazine mesylate

150 mg (0.374 mmol) of 2-(1-(4-(4-methylpiperazin-1-yl)phenyl)ethyl)-10H-phenothiazine was dissolved in 2 mL of ethyl acetate at 20-25°C and stirred to be completely dissolved. Then 2mL of a mixed solution of ethyl acetate and methanesulfonic acid (36.0mg, 0.374mmol, 1.0eq) was added dropwise to the reaction solution. A large amount of flocculent solids were precipitated during the dropwise addition process. The reaction solution was stirred at 20-25°C for 1h and then filtered. The filter cake as a viscous paste was collected and dried to obtain 90.15 mg of 2-(1-(4-(4-methylpiperazin-1-yl)phenyl)ethyl)-10H-phenothiazine mesylate, with a yield of 48.4%.

Example 19: Preparation of 2-(1-(4-(4-methylpiperazin-1-yl)phenyl)ethyl)-10H-phenothiazine p-toluenesulfonate

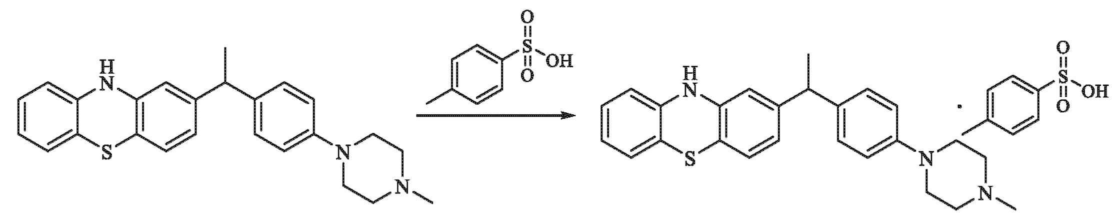

2-(1-(4-(4-methylpiperazin-1-yl)phenyl)ethyl)-10H-phenothiazine

2-(1-(4-(4-methylpiperazin-1-yl)phenyl)ethyl)-10H-phenothiazine p-toluenesulfonate

150 mg (0.374 mmol) of 2-(1-(4-(4-methylpiperazin-1-yl)phenyl)ethyl)-10H-phenothiazine was dissolved in 2 mL of ethyl acetate at 20-25°C and stirred to be completely dissolved. Then 65 mg of p-toluenesulfonic acid (0.374 mmol, 1.0eq) was added to the reaction solution, and a small amount of solids were precipitated. The reaction solution was stirred at 20-25°C for 1h and then filtered. The filter cake as a viscous paste was collected and dried to obtain 100.20 mg of 2-(1-(4-(4-methylpiperazin-1-yl)phenyl)ethyl)-10H-phenothiazine p-toluenesulfonate, with a yield of 46.5%.

Example 20: Preparation of 2-(1-(4-(4-methylpiperazin-1-yl)phenyl)ethyl)-10H-phenothiazine citrate

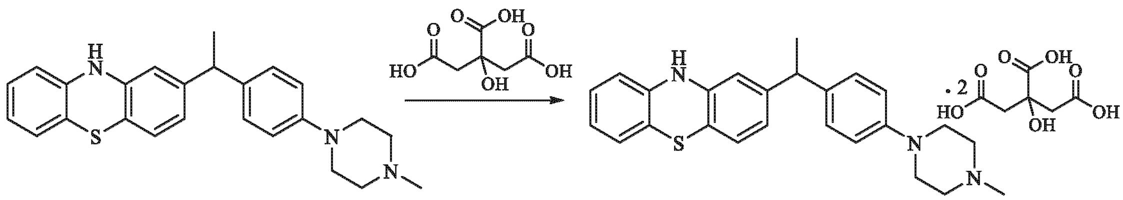

2-(1-(4-(4-methylpiperazin-1-yl)phenyl)ethyl)-10H-phenothiazine

2-(1-(4-(4-methylpiperazin-1-yl)phenyl)ethyl)-10H-phenothiazine cirate

Method 1

150 mg (0.374 mmol) of 2-(1-(4-(4-methylpiperazin-1-yl)phenyl)ethyl)-10R-phenothiazine was dissolved in 4 mL of methanol at 20-25°C and stirred to be completely dissolved. A solid was slowly precipitated after dissolution. Subsequently, the reaction solution was added with 72 mg of citric acid (0.374 mmol, 1.0eq), which became clear from cloudy, and then a solid was gradually precipitated. The reaction system was stirred at 20-25°C for 4 h and filtered. The filter cake was dried to obtain 183.10 mg of 2-(1 -(4-(4-methylpiperazin-1-yl)phenyl)ethyl)-10H-phenothiazine citrate.

Method 2

150 mg (0.374 mmol) of 2-(1-(4-(4-methylpiperazin-1-yl)phenyl)ethyl)-10R-phenothiazine was dissolved in 4 mL of acetone at 20-25°C and stirred to be completely dissolved. Subsequently, the reaction solution was added with 72 mg of citric acid (0.374 mmol, 1.0eq), stirred, and a solid was gradually precipitated during stirring. The mixture was stirred at 20-25°C for 2 h, and then the reaction system was filtered. The filter cake was dried to obtain 179.63 mg of 2-(1-(4-(4-methylpiperazin-1-yl)phenyl)ethyl)-10H-phenothiazine citrate. $^1$H NMR (400 MHz, DMSO-d6) δ=10.88 (s, 2H), 8.51 (s, 1H), 7.09 (d, J = 8.6 Hz, 2H), 6.99 - 6.93 (m, 1H), 6.89 (d, J = 8.4 Hz, 3H), 6.82 (d, J = 7.6 Hz, 1H), 6.73 (m, 1H), 6.66 (m, 2H), 6.52 (d, J = 1.6 Hz, 1H), 3.91 (m, 1H), 3.22 (s, 4H), 2.92 (d, J = 4.4 Hz, 4H), 2.65 (d, J = 15.2 Hz, 2H), 2.57 (m, 4H), 2.53 (s, 2H), 2.09 (s, 3H), 1.47 (d, J = 7.2 Hz, 3H), (See FIG. 12 for $^1$H NMR spectra). It was confirmed though structure analysis that 2-(1-(4-(4-methylpiperazin-1-yl)phenyl)ethyl)-10H-phenothiazine was combined with citric acid at 1:2 in the resulting citrate salt.

Method 3

150 mg (0.374 mmol) of 2-(1-(4-(4-methylpiperazin-1-yl)phenyl)ethyl)-10H-phenothiazine was dissolved in 2 mL of ethyl acetate at 20-25°C and stirred to be completely dissolved. Subsequently, the reaction solution was added with 72 mg of citric acid (0.374 mmol, 1.0eq), stirred, and a solid was gradually precipitated during stirring. The mixture was stirred at 20-25°C for 4 h, and then the reaction system was filtered. The filter cake was dried to obtain 189.4 mg of 2-(1 -(4-(4-methylpiperazin-1-yl)phenyl)ethyl)-10H-phenothiazine citrate.

The impurities of the above citrate salt product were detected and the total impurity content was 0.64% to 0.80%.

Example 21: Preparation of 2-(1-(4-(4-methylpiperazin-1-yl)phenyl)ethyl)-10H-phenothiazine fumarate

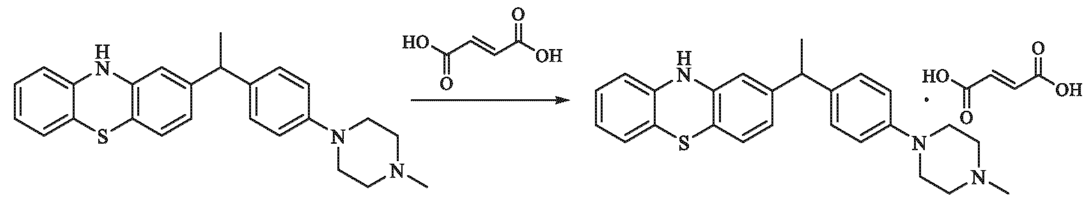

2-(1-(4-(4-methylpiperazin-1-yl)phenyl)ethyl)-10H-phenothiazine

2-(1-(4-(4-methylpiperazin-1-yl)phenyl)ethyl)-10H-phenothiazine fumarate

Method 1

150 mg (0.374 mmol) of 2-(1-(4-(4-methylpiperazin-1-yl)phenyl)ethyl)-10H-phenothiazine was dissolved in 2 mL of ethyl acetate at 20-25°C and stirred to be completely dissolved. Then the reaction solution was added with 43.41 mg of fumaric acid (trans-butenedioic acid; 0.374 mmol, 1.0 eq) and stirred, and a solid was gradually precipitated during stirring. The mixture was stirred at 20-25°C for 4-5 h, and then a large amount of solid was precipitated. The reaction system was filtered. The filter cake was dried to obtain 146.3 mg of 2-(1-(4-(4-methylpiperazin-1-yl)phenyl)ethyl)-10H-phenothiazine fumarate with a yield of 75.8%. $^1H$ NMR (400 MHz, DMSO-d6) δ=8.51 (s, 1H), 7.06 (d, J = 8.6 Hz, 2H), 6.99 - 6.93 (m, 1H), 6.91 - 6.84 (m, 3H), 6.81 (d, J = 8.0 Hz, 1H), 6.73 (m, 1H), 6.65 (m, 2H), 6.59 (s, 2H), 6.51 (d, J = 1.6 Hz, 1H), 3.90 (m, 1H), 3.22 - 3.08 (m, 4H), 2.70 - 2.58 (m, 4H), 2.35 (s, 3H), 1.46 (d, J = 7.2 Hz, 3H), (See FIG. 13 for $^1H$ NMR spectra). It was confirmed though structure analysis that 2-(1-(4-(4-methylpiperazin-1-yl)phenyl)ethyl)-10H-phenothiazine was combined with fumaric acid at 1:1 in the resulting fumarate salt.

Method 2

150 mg (0.374 mmol) of 2-(1-(4-(4-methylpiperazin-1-yl)phenyl)ethyl)-10R-phenothiazine was dissolved in 2 mL of acetone at 20-25°C and stirred to be completely dissolved. Then, 43.41 mg of fumaric acid (trans-butenedioic acid; 0.374 mmol, 1.0 eq) was added to the reaction solution. At first a small amount of solid was precipitated at the bottom of the reaction flask, and then it gradually became an oil substance. The mixture was stirred at 20-25°C for 4-5 h, and then a large amount of solid was precipitated. The reaction system was filtered. The filter cake was dried to obtain 121 mg of 2-(1-(4-(4-methylpiperazin-1-yl)phenyl)ethyl)-10H-phenothiazine fumarate with a yield of 62.5%.

The impurities of the above fumarate salt product were detected and the total impurity content was 0.76% to 0.84%.

Example 22: Preparation of 2-(1-(4-(4-methylpiperazin-1-yl)phenyl)ethyl)-10H-phenothiazine tartrate

Method 1

150 mg (0.374 mmol) of 2-(1-(4-(4-methylpiperazin-1-yl)phenyl)ethyl)-10R-phenothiazine was dissolved in 2 mL of acetone at 20-25°C and stirred to be completely dissolved. Then, 56 mg of tartaric acid (DL type; 0.374 mmol, 1.0 eq) was added to the reaction solution, and stirred. A small amount of solid was precipitated at the bottom of the reaction flask during stirring. The mixture was stirred at 20-25°C for 2 h, and then a solid was precipitated. The reaction system was filtered. The filter cake was dried to obtain 155.3 mg of 2-(1-(4-(4-methylpiperazin-1-yl)phenyl)ethyl)-10R-phenothiazine tartrate.

Method 2

150 mg (0.374 mmol) of 2-(1-(4-(4-methylpiperazin-1-yl)phenyl)ethyl)-10H-phenothiazine was dissolved in 2 mL of ethyl acetate at 20-25°C and stirred to be completely dissolved. Then, 56 mg of tartaric acid (DL type; 0.374 mmol, 1.0 eq) was added to the reaction solution and stirred. A small amount of solid was precipitated in the bottom of the reaction flask during stirring. The mixture was stirred at 20-25°C for 2 h, and then a large amount of solid was precipitated. The reaction system was filtered. The filter cake was dried to obtain 166.5 mg of 2-(1-(4-(4-methylpiperazin-1-yl)phenyl) ethyl)-10H-phenothiazine tartrate.

Example 23: Preparation of 2-(1-(4-(4-methylpiperazin-1-yl)phenyl)ethyl)-10H-phenothiazine maleate

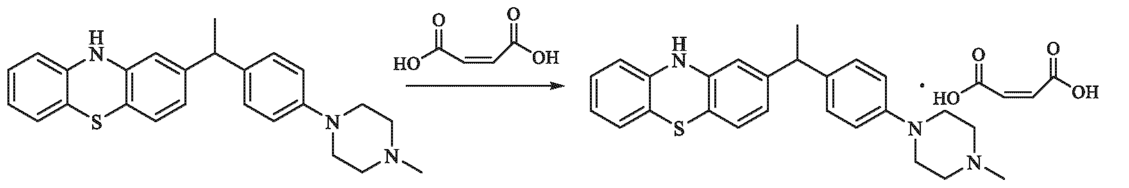

2-(1-(4-(4-methylpiperazin-1-yl)phenyl)ethyl)-10H-phenothiazine

2-(1-(4-(4-methylpiperazin-1-yl)phenyl)ethyl)-10H-phenothiazine maleate

150 mg (0.374 mmol) of 2-(1-(4-(4-methylpiperazin-1-yl)phenyl)ethyl)-10H-phenothiazine was dissolved in 3 mL of ethyl acetate at 20-25°C and stirred to be completely dissolved. Then, 43.41 mg of maleic acid (cis-butenedioic acid; 0.374 mmol, 1.0 eq) was added to the reaction solution and stirred. A small amount of solid was precipitated at the bottom of the reaction flask during stirring, and then it gradually became an oil substance. The mixture was stirred at 20-25°C for 4-5 h, and then a large amount of solid was precipitated. The reaction system was filtered. The filter cake was dried to obtain 132.8 mg of 2-(1-(4-(4-methylpiperazin-1-yl)phenyl)ethyl)-10H-phenothiazine maleate, with a yield of 68.6%, and a total impurity content of 0.42%. $^1$H NMR (400 MHz, DMSO-d6) δ=8.51 (s, 1H), 7.11 (d, J = 8.8 Hz, 2H), 7.00 - 6.87 (m, 4H), 6.82 (d, J = 8.0 Hz, 1H), 6.73 (m, 1H), 6.66 (dd, J = 8.0, 2.0 Hz, 2H), 6.54 (d, J = 1.6 Hz, 1H), 6.05 (s, 2H), 3.92 (m, 1H), 3.28 (s, 8H), 2.83 (s, 3H), 1.47 (d, J = 7.2 Hz, 3H), (See FIG. 14 for $^1$H NMR spectra). It was confirmed though structure analysis that 2-(1-(4-(4-methylpiperazin-1-yl)phenyl)ethyl)-10H-phenothiazine was combined with maleic acid at 1:1 in the resulting maleate salt.

Example 24: Preparation of 2-(1-(4-(4-methylpiperazin-1-yl)phenyl)ethyl)-10H-phenothiazine hydrobromide

Referring to the preparation method in Example 17, 2-(1-(4-(4-methylpiperazin-1-yl)phenyl)ethyl)-10H-phenothiazine hydrobromide was prepared by replacing hydrochloric acid with hydrobromic acid.

Example 25: Preparation of 2-(1-(4-(4-methylpiperazin-1-yl)phenyl)ethyl)-10H-phenothiazine phosphate

Referring to the preparation method in Example 17, 2-(1-(4-(4-methylpiperazin-1-yl)phenyl)ethyl)-10H-phenothiazine phosphate was prepared by replacing hydrochloric acid with phosphoric acid.

Example 26: Preparation of 2-(1-(4-(4-methylpiperazin-1-yl)phenyl)ethyl)-10H-phenothiazine oxalate

Referring to the preparation method in Example 17, 2-(1-(4-(4-methylpiperazin-1-yl)phenyl)ethyl)-10H-phenothiazine oxalate was prepared by replacing hydrochloric acid with oxalic acid.

Example 27: Preparation of 2-(1-(4-(4-methylpiperazin-1-yl)phenyl)ethyl)-10H-phenothiazine esylate

Referring to the preparation method in Example 21, 2-(1-(4-(4-methylpiperazin-1-yl)phenyl)ethyl)-10H-phenothiazine esylate was prepared by replacing fumaric acid with ethanesulfonic acid.

Example 28: Preparation of 2-(1-(4-(4-methylpiperazin-1-yl)phenyl)ethyl)-10H-phenothiazine malate

Referring to the preparation method in Example 21, 2-(1-(4-(4-methylpiperazin-1-yl)phenyl)ethyl)-10H-phenothiazine malate was prepared by replacing fumaric acid with malic acid.

Example 29: Preparation of 2-(1-(4-(4-methylpiperazin-1-yl)phenyl)ethyl)-10H-phenothiazine succinate

Referring to the preparation method in Example 21, 2-(1-(4-(4-methylpiperazin-1-yl)phenyl)ethyl)-10H-phenothiazine succinate was prepared by replacing fumaric acid with butanedioic acid.

Example 30: Preparation of 2-(1-(4-(4-methylpiperazin-1-yl)phenyl)ethyl)-10H-phenothiazine propionate

Referring to the preparation method in Example 21, 2-(1-(4-(4-methylpiperazin-1-yl)phenyl)ethyl)-10H-phenothiazine propionate was prepared by replacing fumaric acid with propionic acid.

Example 31: Comparative study on solubility

According to the solubility measurement method in the General Notices of Chinese Pharmacopoeia (2020 edition), 2-(1-(4-(4-methylpiperazin-1-yl)phenyl)ethyl)-10H-phenothiazine, 2-(1-(4-(4-methylpiperazin-1-yl)phenyl) ethyl)-10H-phenothiazine hydrochloride, 2-(1-(4-(4-methylpiperazin-1-yl)phenyl)ethyl)-10H-phenothiazine citrate, 2-(1 -(4-(4-methylpiperazin-1-yl)phenyl)ethyl)-10H-phenothiazine maleate, and 2-(1-(4-(4-methylpiperazin-1-yl)phenyl) ethyl)-10H-phenothiazine fumarate were added to a certain volume of solvent at 25°C ± 2°C respectively, and were shaken strongly for 30 seconds every 5 minutes. The dissolution phenomena was observed for 30 minutes and the solubility was measured.

Table 25 Data for solubility

| Compound | Solubility | |
|---|---|---|
| | Water | Hydrochloric acid solution at pH 1.0 |
| 2-(1-(4-(4-methylpiperazin-1-yl)phenyl)ethyl)-10H-phenothiazine | <10ng/mL | 0.034mg/mL |
| 2-(1-(4-(4-methylpiperazin-1-yl)phenyl)ethyl)-10H-phenothiazine hydrochloride | 0.077mg/mL | 0.35mg/mL |
| 2-(1-(4-(4-methylpiperazin-1-yl)phenyl)ethyl)-10H-phenothiazine citrate | 0.01mg/mL | NA |
| 2-(1-(4-(4-methylpiperazin-1-yl)phenyl)ethyl)-10H-phenothiazine maleate | <2μ/ml | <2.5μg/ml |
| 2-(1-(4-(4-methylpiperazin-1-yl)phenyl)ethyl)-10H-phenothiazine fumarate | <2μg/ml | <2.5μg/ml |
| NA represents that no measurement was performed. | | |

The results of solubility measurement show that the solubility of 2-(1-(4-(4-methylpiperazin-1-yl)phenyl) ethyl)-10H-phenothiazine hydrochloride was better than that of 2-(1-(4-(4-methylpiperazin-1-yl)phenyl)ethyl)-10H-phenothiazine in both water and hydrochloric acid solution at pH 1.0, especially in water. The solubility of 2-(1-(4-(4-methylpiperazin-1-yl)phenyl)ethyl)-10H-phenothiazine citrate in water was 1000 times greater than that of 2-(1-(4-(4-methylpiperazin-1-yl)phenyl)ethyl)-10R-phenothiazine.

In addition, the solubility of the hydrochloride salt was also significantly better compared to the solubility of the other salts of the compound, wherein the solubility of the hydrochloride salt was 7 times higher than that of the citrate salt with a good solubility, and more than 35 times higher than that of the maleate and fumarate salts.

Example 32: Stability test 1

Test (1): High temperature test

According to the method of 9001 'Guidelines for Stability Tests of API Drugs and Preparations' in the Chinese Pharmacopoeia (2020 Edition), the test substance was placed in a suitable constant temperature equipment at 60°C, the samples were taken on days 5, 10 and 30, respectively.

The results of high-temperature test show that the salts of 2-(1-(4-(4-methylpiperazin-1-yl)phenyl)ethyl)-10H-phenothiazine was more stable than free 2-(1-(4-(4-methylpiperazin-1-yl)phenyl)ethyl)-10H-phenothiazine at high temperature condition. The hydrochloride salt thereof had no change in the properties and appearance, and generated a low content of total impurity, indicating that it has the best stability.

Table 26 Data for stability at high temperature condition

| Condition | | High temperature (60°C) (Amount of impurity at a level greater than the reporting limit) | | | |
|---|---|---|---|---|---|
| Collection time point | | Day 0 | Day 5 | Day 10 | Day 30 |
| Compound | 2-(1-(4-(4-methylpiperazin-1-yl)phenyl) ethyl)-10H -phenothiazine | 9 | - | - | - |
| | 2-(1-(4-(4-methylpiperazin-1-yl)phenyl) ethyl)-10H -phenothiazine hydrochloride | 2 | 2 | 2 | 2 |
| | 2-(1-(4-(4-methylpiperazin-1-yl)phenyl) ethyl)-10H -phenothiazine citrate | 7 | 7 | 8 | 8 |
| | 2-(1-(4-(4-methylpiperazin-1-yl)phenyl) ethyl)-10H -phenothiazine fumarate | 8 | 8 | 8 | 8 |
| | 2-(1-(4-(4-methylpiperazin-1-yl)phenyl) ethyl)-10H -phenothiazine maleate | 5 | 5 | 5 | 5 |

Test (2): High humidity test

According to 9001 'Guideline for Stability Tests of API Drugs and Preparations' in Chinese Pharmacopoeia (2020 edition), the test substance was placed in a constant humidity airtight container at 25°C under a relative humidity of 90% ±5% for 30 days, and the samples were taken on days 5, 10, and 30.

The results of high humidity test show that, the salts of 2-(1-(4-(4-methylpiperazin-1-yl)phenyl)ethyl)-10H-phenothiazine had a stability higher than that of the free 2-(1-(4-(4-methylpiperazin-1-yl)phenyl)ethyl)-10H-phenothiazine under high humidity condition. The hydrochloride salt thereof had a better stability compared to other salts under high humidity, had no changes in properties and appearance, and generated a low content of total impurity.

Table 27 Data for stability under high humidity condition

| Condition | | High humidity (Relative humidity 90%±5%) (Amount of impurity at a level greater than the reporting limit) | | | |
|---|---|---|---|---|---|
| Collection time point | | Day 0 | Day 5 | Day 10 | Day 30 |
| Compound | 2-(1-(4-(4-methylpiperazin-1-yl)phenyl) ethyl)-10H -phenothiazine | 9 | - | - | - |
| | 2-(1-(4-(4-methylpiperazin-1-yl)phenyl) ethyl)-10H -phenothiazine hydrochloride | 2 | 2 | 2 | 2 |
| | 2-(1-(4-(4-methylpiperazin-1-yl)phenyl) ethyl)-10H -phenothiazine citrate | 7 | 6 | 8 | 6 |
| | 2-(1-(4-(4-methylpiperazin-1-yl)phenyl) ethyl)-10H -phenothiazine fumarate | 8 | 8 | 8 | 8 |
| | 2-(1-(4-(4-methylpiperazin-1-yl)phenyl) ethyl)-10H -phenothiazine maleate | 5 | 5 | 5 | 5 |

Test (3): Illumination test

According to the 9001 'Guidelines for Stability Tests of APIs and Preparations' in Chinese Pharmacopoeia (2020 edition), the test substance was placed in a stability test chamber under a condition of illuminance of 45001x±5001x, a total illuminance of the light source not less than $1.2\times10^6$lux• hr and near-ultra violet light energy not less than 200W•hr/$m^2$ for 30 days. The samples were taken on days 5, 10, and 30.

The results of the illumination test show that, the salts of 2-(1-(4-(4-methylpiperazin-1-yl)phenyl)ethyl)-10H-phenothiazine were more stable than free 2-(1-(4-(4-methylpiperazin-1-yl)phenyl)ethyl)-10H-phenothiazine under the illumination condition. Wherein, the citrate salt was changed from light yellow to grey after 5 days of illumination, and then turned to dark grey after 10 days of illumination. The hydrochloride salt had a better stability compared to other salts under the illumination condition, with no change in properties and appearance, and a low content of total impurity.

Table 28 Data for stability under illumination condition

| Condition | | Illumination (illuminance of 45001x±5001x) (Amount of impurity at a level greater than the reporting limit) | | | |
|---|---|---|---|---|---|
| Collection time point | | Day 0 | Day 5 | Day 10 | Day 30 |
| Compound | 2-(1-(4-(4-methylpiperazin-1-yl)phenyl) ethyl)-10H -phenothiazine | 9 | - | - | - |
| | 2-(1-(4-(4-methylpiperazin-1-yl)phenyl) ethyl)-10H -phenothiazine hydrochloride | 2 | 2 | 2 | 2 |
| | 2-(1-(4-(4-methylpiperazin-1-yl)phenyl) ethyl)-10H -phenothiazine citrate | 7 | 7 | 9 | 10 |
| | 2-(1-(4-(4-methylpiperazin-1-yl)phenyl) ethyl)-10H -phenothiazine fumarate | 8 | 9 | 8 | 7 |
| | 2-(1-(4-(4-methylpiperazin-1-yl)phenyl) ethyl)-10H-phenothiazine maleate | 5 | 5 | 5 | 5 |

In summary, the results of the stability test show that, compared to the corresponding free compound and other salts thereof, the stability of 2-(1-(4-(4-methylpiperazin-1-yl)phenyl)ethyl)-10H-phenothiazine hydrochloride was higher at high temperature and under high humidity and illumination conditions, and its total impurity content was significantly lower.

Example 33: Stability test 2

The same mass of 2-(1-(4-(4-methylpiperazin-1-yl)phenyl)ethyl)-10H-phenothiazine hydrochloride, 2-(1-(4-(4-methylpiperazin-1-yl)phenyl)ethyl)-10H-phenothiazine citrate, 2-(1-(4-(4-methylpiperazin-1-yl)phenyl)ethyl)-10H-phenothiazine fumarate, and 2-(1-(4-(4-methylpiperazin-1-yl)phenyl)ethyl)-10H-phenothiazine maleate, was weighed and added to a certain volume of hydrochloric acid solution of pH 1.0 (roughly the acidity of human gastric liquid) at 25°C ± 2°C. The solution was shaken strongly for 30 seconds every 5 minutes, and the color and status of the solution was observed at 0.5h and 24h.

Table 29 Data for stability test 2

| Compound | Hydrochloric acid solution of pH 1.0 | |
|---|---|---|
| | 0.5h | 24h |
| 2-(1-(4-(4-methylpiperazin-1-yl)phenyl)ethyl)-10H -phenothiazine hydrochloride (gray-white) | Colorless transparent solution | Colorless transparent solution |
| 2-(1-(4-(4-methylpiperazin-1-yl)phenyl)ethyl)-10H -phenothiazine citrate (off-white) | Light pink solution | Pink solution |
| 2-(1-(4-(4-methylpiperazin-1-yl)phenyl)ethyl)-10H -phenothiazine fumarate (off-white) | Light pink solution | Pink solution |
| 2-(1-(4-(4-methylpiperazin-1-yl)phenyl)ethyl)-10H -phenothiazine maleate (off-white) | Light pink solution | Pink solution |

The experimental results showed that the respective solution of 2-(1-(4-(4-methylpiperazin-1-yl)phenyl) ethyl)-10H-phenothiazine citrate, 2-(1-(4-(4-methylpiperazin-1-yl)phenyl)ethyl)-10H-phenothiazine fumarate, and 2-(1-(4-(4-methylpiperazin-1-yl)phenyl)ethyl)-10H-phenothiazine maleate in hydrochloric acid solution of pH 1.0 showed significant changes in solution color, indicating that their stability was poorer than that of 2-(1-(4-(4-methylpiperazin-1-yl) phenyl)ethyl)-10H-phenothiazine hydrochloride.

Example 34: Hygroscopicity test

In accordance with the Guidelines for Hygroscopicity of Drugs of Part IV of the Chinese Pharmacopoeia (2020 Edition), the salts obtained in above examples, such as 2-(1-(4-(4-methylpiperazin-1-yl)phenyl)ethyl)-10H-phenothiazine, 2-(1-(4-(4-methylpiperazin-1-yl)phenyl)ethyl)-10H-phenothiazine hydrochloride, and 2-(1-(4-(4-methylpiperazine-1-yl)phenyl)ethyl)-10H-phenothiazine citrate, were subjected to hygroscopicity test.

The description of the characteristics of hygroscopicity and the definition of the weight gain due to hygroscopicity are detailed as follows:

Deliquescence: sufficient water is absorbed to form a liquid.

Very hygroscopic: the weight gain due to hygroscopicity is not less than 15%.

Hygroscopic: the weight gain due to hygroscopicity is less than 15% but not less than 2%.

Slightly hygroscopic: the weight gain due to hygroscopicity is less than 2% but not less than 0.2%.

Not or nearly not hygroscopic: the weight gain due to hygroscopicity is less than 0.2%.

The experimental results show that 2-(1-(4-(4-methylpiperazin-1-yl)phenyl)ethyl)-10H-phenothiazine hydrochloride was slightly hygroscopic, and it had excellent physicochemical properties and the best druggability.

The examples described above are merely for explaining the technical concept and the features of the present disclosure, and aim to enable the person skilled in the art to understand the contents of the present disclosure and implement the same accordingly, but the scope of the present disclosure is not limited thereto. All equivalent changes or modifications made according to the spirit of the present disclosure should be covered within the protection scope of the present disclosure.

## Claims

1. A crystal Form A of 2-(1-(4-(4-methylpiperazin-1-yl)phenyl)ethyl)-10R-phenothiazine hydrochloride, wherein the X-ray powder diffraction pattern with Cu-K$\alpha$ radiation of the crystal Form A comprises characteristic peaks at 20 of 10.68 $\pm$0.2°, 14.36$\pm$0.2°, 18.57$\pm$0.2°, 21.08$\pm$0.2°, 22.14$\pm$0.2°, 23.40$\pm$0.2°, and 29.03$\pm$0.2°;

   preferably, the X-ray powder diffraction pattern with Cu-K$\alpha$ radiation of the crystal Form A comprises characteristic peaks at 20 of 10.68$\pm$0.2°, 14.36$\pm$0.2°, 17.84$\pm$0.2°, 18.57$\pm$0.2°, 21.08$\pm$0.2°, 22.14$\pm$0.2°, 23.40$\pm$0.2°, 27.50$\pm$0.2°, and 29.03$\pm$0.2°;
   preferably, the X-ray powder diffraction pattern with Cu-K$\alpha$ radiation of the crystal Form A comprises characteristic peaks at 20 of 10.68$\pm$0.2°, 14.36$\pm$0.2°, 16.54$\pm$0.2°, 17.84$\pm$0.2°, 18.57$\pm$0.2°, 20.89$\pm$0.2°, 21.08$\pm$0.2°, 22.14$\pm$0.2°, 22.92$\pm$0.2°, 23.40$\pm$0.2°, 25.88$\pm$0.2°, 27.50$\pm$0.2°, and 29.03$\pm$0.2°;
   preferably, the X-ray powder diffraction pattern with Cu-K$\alpha$ radiation of the crystal Form A comprises characteristic peaks at 20 of 10.68$\pm$0.2°, 12.63$\pm$0.2°, 14.36$\pm$0.2°, 16.06$\pm$0.2°, 16.54$\pm$0.2°, 17.84$\pm$0.2°, 18.57$\pm$0.2°, 20.89$\pm$0.2°, 21.08$\pm$0.2°, 22.14$\pm$0.2°, 22.92$\pm$0.2°, 23.40$\pm$0.2°, 25.11$\pm$0.2°, 25.51$\pm$0.2°, 25.88$\pm$0.2°, 27.50 $\pm$0.2°, 28.54$\pm$0.2°, 29.03$\pm$0.2°, and 33.55$\pm$0.2°; and
   more preferably, the X-ray powder diffraction pattern with Cu-K$\alpha$ radiation of the crystal Form A is shown in FIG. 1.

2. A crystal Form B of 2-(1-(4-(4-methylpiperazin-1-yl)phenyl)ethyl)-10R-phenothiazine hydrochloride, wherein the X-ray powder diffraction pattern with Cu-K$\alpha$ radiation of the crystal Form B comprises characteristic peaks at 20 of 16.46 $\pm$0.2°, and 22.01$\pm$0.2°;

   preferably, the X-ray powder diffraction pattern with Cu-K$\alpha$ radiation of the crystal Form B comprises characteristic peaks at 20 of 16.46$\pm$0.2°, 22.01$\pm$0.2°, 27.62$\pm$0.2°, 28.91$\pm$0.2°, and 33.41$\pm$0.2°;
   preferably, the X-ray powder diffraction pattern with Cu-K$\alpha$ radiation of the crystal Form B comprises characteristic peaks at 20 of 5.41$\pm$0.2°, 10.92$\pm$0.2°, 16.46$\pm$0.2°, 22.01$\pm$0.2°, 27.62$\pm$0.2°, 28.91$\pm$0.2°, and 33.41$\pm$0.2°; and
   more preferably, the X-ray powder diffraction pattern with Cu-K$\alpha$ radiation of the crystal Form B is shown in FIG. 3.

3. A pharmaceutical composition comprising the crystal Form A according to claim 1, or the crystal Form B according to claim 2, and a pharmaceutically acceptable carrier.

4. Use of the crystal Form A according to claim 1, the crystal Form B according to claim 2, or the pharmaceutical composition according to claim 3 in the manufacture of a medicament for preventing and/or treating a disease selected from the group consisting of a cancer, organ damage, and degenerative disease.

5. Use of the crystal Form A according to claim 1, the crystal Form B according to claim 2, or the pharmaceutical composition according to claim 3 in the manufacture of a medicament for preventing and/or treating a disease selected from the group consisting of a cancer, neurodegenerative disease, cardiovascular and cerebrovascular disease, immune-related disease, liver and kidney failure, inflammation, and metabolic disease;

   preferably, the disease is selected from the group consisting of a cancer, Alzheimer's disease, Parkinson's disease, multiple sclerosis, Huntington's chorea, amyotrophic lateral sclerosis, stroke, ischemia-reperfusion injury, atherosclerosis, immune-related disease, liver and kidney failure, inflammation, diabetes, and diabetic complications; and
   preferably, the stroke is hemorrhagic stroke and/or ischemic stroke.

6. Use of the crystal Form A according to claim 1, the crystal Form B according to claim 2, or the pharmaceutical composition according to claim 3 in the manufacture of a ferroptosis inhibitor.

7. A method for producing the crystal Form A according to claim 1, comprising dispersing a compound of 2-(1-(4-(4-methylpiperazin-1-yl)phenyl)ethyl)-10H-phenothiazine in methanol at 10°C to 40°C, followed by dropwise adding a mixed solution of concentrated hydrochloric acid and methanol, stirring for crystallization, filtering, washing a filter cake with methanol, and drying the filter cake under vacuum to obtain a crystal substance;

   preferably, a weight ratio of the methanol as a solvent to the compound of 2-(1-(4-(4-methylpiperazin-1-yl)phenyl)ethyl)-10H-phenothiazine is 5:1-15:1, preferably 8:1-12:1;
   preferably, a weight ratio of the compound of 2-(1-(4-(4-methylpiperazin-1-yl)phenyl)ethyl)-10H-phenothiazine to the concentrated hydrochloric acid is 1:1-8:1, preferably 3:1-5:1; and
   preferably, the stirring for crystallization is performed for 1h to 8h, preferably for 2h to 5h.

8. A method for producing the crystal Form A according to claim 1, comprising dispersing a compound of 2-(1-(4-(4-methylpiperazin-1-yl)phenyl)ethyl)-10H-phenothiazine in acetone and water at 10°C to 40°C, followed by dropwise adding a mixed solution of concentrated hydrochloric acid, acetone and water, stirring for crystallization, filtering, washing a filter cake with acetone, and drying the filter cake under vacuum to obtain a crystal substance;

   preferably, a weight ratio of the acetone and water as solvent to the compound of 2-(1-(4-(4-methylpiperazin-1-yl)phenyl)ethyl)-10H-phenothiazine is (1-8):(0.5-3):(0.5-3), preferably (4-6):(0.5-1):(0.5-1);
   preferably, a weight ratio of the compound of 2-(1-(4-(4-methylpiperazin-1-yl)phenyl)ethyl)-10H-phenothiazine to the concentrated hydrochloric acid is 1:1-8:1, preferably 3:1-5:1; and
   preferably, the stirring for crystallization is performed for 1h to 8h, preferably for 2h to 5h.

9. A salt of 2-(1-(4-(4-methylpiperazin-1-yl)phenyl)ethyl)-10H-phenothiazine selected from the group consisting of:

   2-(1-(4-(4-methylpiperazin-1-yl)phenyl)ethyl)-10H-phenothiazine hydrochloride;
   2-(1-(4-(4-methylpiperazin-1-yl)phenyl)ethyl)-10H-phenothiazine mesylate;
   2-(1-(4-(4-methylpiperazin-1-yl)phenyl)ethyl)-10H-phenothiazine esylate;
   2-(1-(4-(4-methylpiperazin-1-yl)phenyl)ethyl)-10H-phenothiazine p-toluenesulfonate;
   2-(1-(4-(4-methylpiperazin-1-yl)phenyl)ethyl)-10H-phenothiazine citrate;
   2-(1-(4-(4-methylpiperazin-1-yl)phenyl)ethyl)-10H-phenothiazine fumarate;
   2-(1-(4-(4-methylpiperazin-1-yl)phenyl)ethyl)-10H-phenothiazine maleate;
   2-(1-(4-(4-methylpiperazin-1-yl)phenyl)ethyl)-10H-phenothiazine tartrate;
   2-(1-(4-(4-methylpiperazin-1-yl)phenyl)ethyl)-10H-phenothiazine hydrobromide;
   2-(1-(4-(4-methylpiperazin-1-yl)phenyl)ethyl)-10H-phenothiazine oxalate;
   2-(1-(4-(4-methylpiperazin-1-yl)phenyl)ethyl)-10H-phenothiazine phosphate;
   2-(1-(4-(4-methylpiperazin-1-yl)phenyl)ethyl)-10H-phenothiazine sulphate;
   2-(1-(4-(4-methylpiperazin-1-yl)phenyl)ethyl)-10H-phenothiazine acetate;
   2-(1-(4-(4-methylpiperazin-1-yl)phenyl)ethyl)-10H-phenothiazine propionate;
   2-(1-(4-(4-methylpiperazin-1-yl)phenyl)ethyl)-10H-phenothiazine perchlorate;
   2-(1-(4-(4-methylpiperazin-1-yl)phenyl)ethyl)-10H-phenothiazine malate;
   2-(1-(4-(4-methylpiperazin-1-yl)phenyl)ethyl)-10H-phenothiazine salicylate;
   2-(1-(4-(4-methylpiperazin-1-yl)phenyl)ethyl)-10H-phenothiazine mandelate;
   2-(1-(4-(4-methylpiperazin-1-yl)phenyl)ethyl)-10H-phenothiazine lactate; and
   2-(1-(4-(4-methylpiperazin-1-yl)phenyl)ethyl)-10H-phenothiazine succinate.

**10.** The salt according to claim 9, wherein the salt is 2-(1-(4-(4-methylpiperazin-1-yl)phenyl)ethyl)-10H-phenothiazine hydrochloride.

**11.** The salt according to claim 9, wherein a molar ratio of 2-(1-(4-(4-methylpiperazin-1-yl)phenyl)ethyl)-10H-phenothiazine to hydrochloric acid in the 2-(1-(4-(4-methylpiperazin-1-yl)phenyl)ethyl)-10H-phenothiazine hydrochloride is 1:(0.5-2); preferably, the molar ratio is 1:1 or 1:2.

**12.** A method for producing the salt according to claim 9, comprising performing a reaction of 2-(1-(4-(4-methylpiperazin-1-yl)phenyl)ethyl)-10H-phenothiazine and an acid to form a salt,

preferably, the reaction is carried out in water and/or an organic solvent, and the organic solvent is selected from the group consisting of a ketone with 2 to 6 carbon atoms, ethyl acetate, lower fatty alcohol, tetrahydrofuran and a combination thereof; further, the ketone with 2 to 6 carbon atoms is preferably acetone; still further, the lower fatty alcohol is optionally selected from the group consisting of methanol, ethanol, propanol and isopropanol;
preferably, the reaction is carried out in an solvent selected from the group consisting of acetone, methanol, ethanol, and a combination thereof;
preferably, the acid is selected from the group consisting of hydrochloric acid, HCl, methanesulfonic acid, ethanesulfonic acid, p-toluenesulfonic acid, citric acid, fumaric acid, maleic acid, tartaric acid, hydrobromic acid, oxalic acid, phosphoric acid, sulfuric acid, acetic acid, propionic acid, perchloric acid, malic acid, salicylic acid, mandelic acid, lactic acid and succinic acid, and more preferably hydrochloric acid.

**13.** The method according to claim 12 comprising reacting 2-(1-(4-(4-methylpiperazin-1-yl)phenyl)ethyl)-10H-phenothiazine with hydrochloric acid in acetone, ethanol, isopropanol or tetrahydrofuran, stirring, and filtering.

**14.** A pharmaceutical composition comprising the salt according to any one of claims 9 to 12 and one or more pharmaceutically acceptable carriers and/or diluents; preferably, the salt is hydrochloride.

**15.** Use of the salt according to any one of claims 9 to 12 or the pharmaceutical composition according to claim 14 in the manufacture of a medicament for preventing and/or treating a disease selected from the group consisting of a cancer, organ damage and degenerative disease.

**16.** Use of the salt according to claims 9 to 12 or the pharmaceutical composition according to claim 14 in the manufacture of a medicament for preventing and/or treating a disease selected from the group consisting of a cancer, neurodegenerative disease, cardiovascular and cerebrovascular disease, immune-related disease, liver and kidney failure, inflammation and metabolic disease.

**17.** The use according to claim 15, wherein the disease is selected from the group consisting of a cancer, Alzheimer's disease, Parkinson's disease, multiple sclerosis, Huntington's chorea, amyotrophic lateral sclerosis, stroke, ischemia-reperfusion injury, immune-related disease, liver and kidney failure, inflammation, atherosclerosis, diabetes, and diabetic complications.

**18.** The use according to claim 17, wherein the stroke is hemorrhagic stroke and/or ischemic stroke.

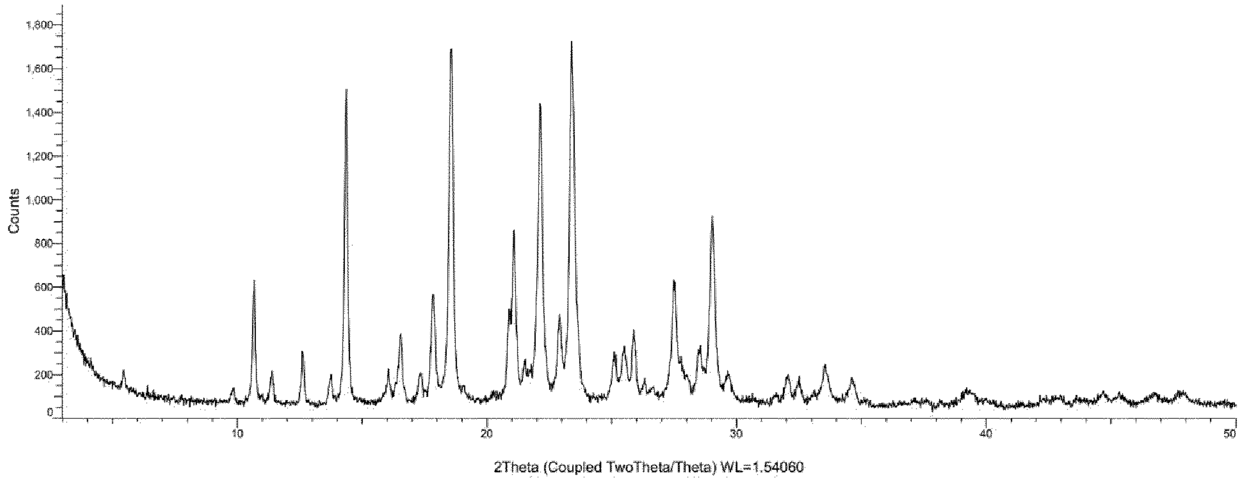
Counts
1,800
1,600
1,400
1,200
1,000
800
600
400
200
0
10
20
30
40
50
2Theta (Coupled TwoTheta/Theta) WL=1.54060

FIG. 1

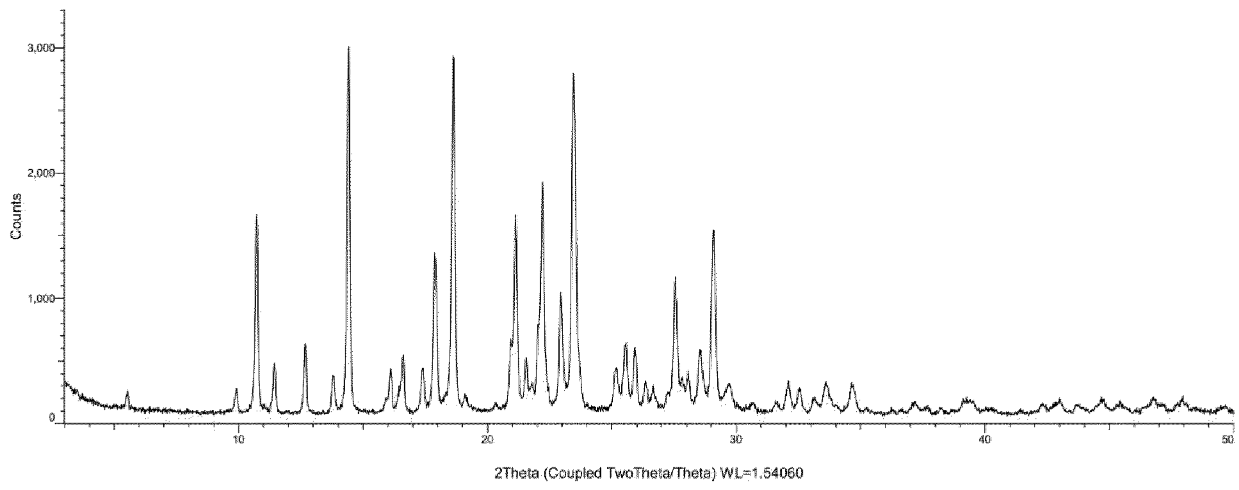
Counts
3,000
2,000
1,000
0
10
20
30
40
50
2Theta (Coupled TwoTheta/Theta) WL=1.54060

FIG. 2

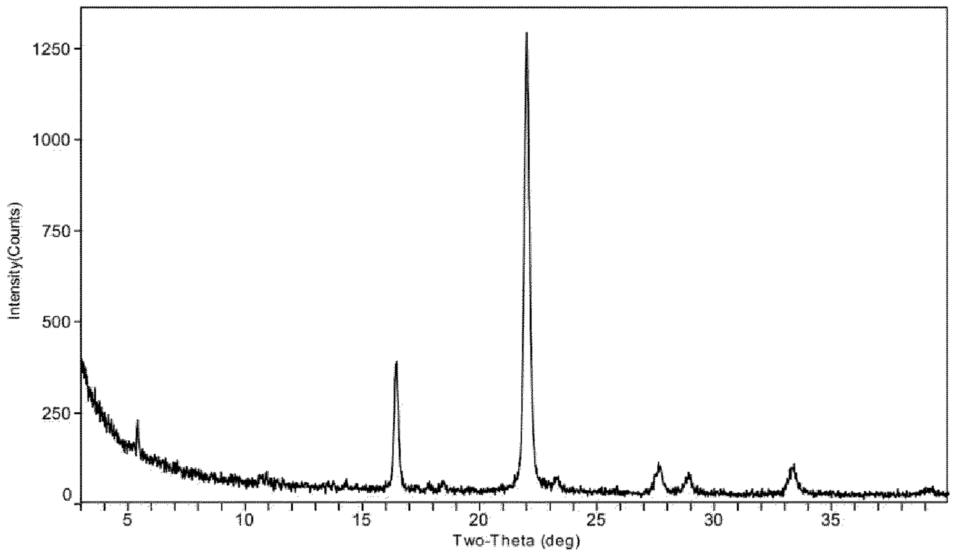


FIG. 3

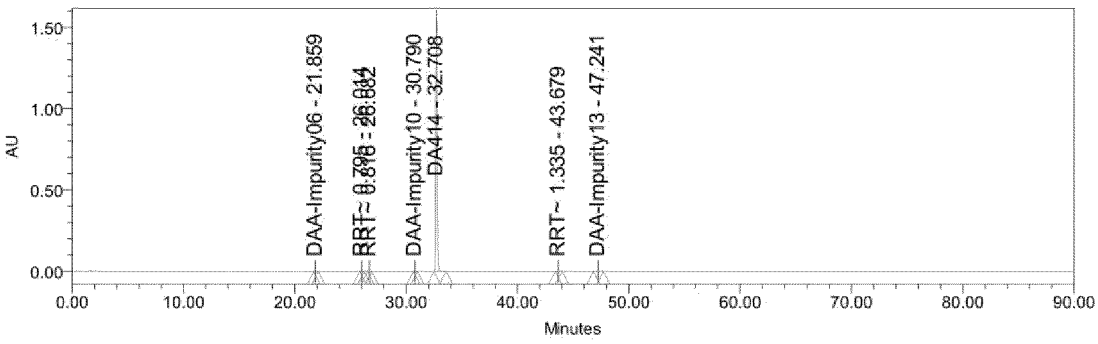


**Peak Results**

| | Name | RT (min) | RRT | Area (¦IV*sec) | % Area | Height (¦IV) | USP Resolution | USP Tailing | USP Plate Count | USP s/n |
|---|---|---|---|---|---|---|---|---|---|---|
| 1 | DAA-Impurity01 | 15.752 | | | | | | | | |
| 2 | DAA-Impurity06 | 21.859 | | 8949 | 0.068 | 1078 | | 1.2 | 151584 | 74 |
| 3 | RRT~ 0.795 | 26.014 | 0.80 | 3033 | 0.023 | 330 | 17.5 | 1.2 | 172972 | 22 |
| 4 | RRT~ 0.816 | 26.682 | 0.82 | 4740 | 0.036 | 555 | 2.9 | 1.3 | 240278 | 38 |
| 5 | DAA-Impurity10 | 30.790 | | 4070 | 0.031 | 460 | 18.1 | 1.1 | 269453 | 31 |
| 6 | DA414 | 32.708 | | 13084195 | 99.764 | 1539327 | 8.4 | 1.2 | 350680 | 107206 |
| 7 | DAA-Impurity12 | 41.159 | | | | | | | | |
| 8 | RRT~ 1.335 | 43.679 | 1.34 | 3953 | 0.030 | 220 | 31.0 | 1.1 | 128629 | 14 |
| 9 | DAA-Impurity13 | 47.241 | | 6209 | 0.047 | 256 | 6.2 | 1.1 | 83582 | 17 |
| 10 | DAA-Impurity16 | 75.466 | | | | | | | | |

FIG. 4

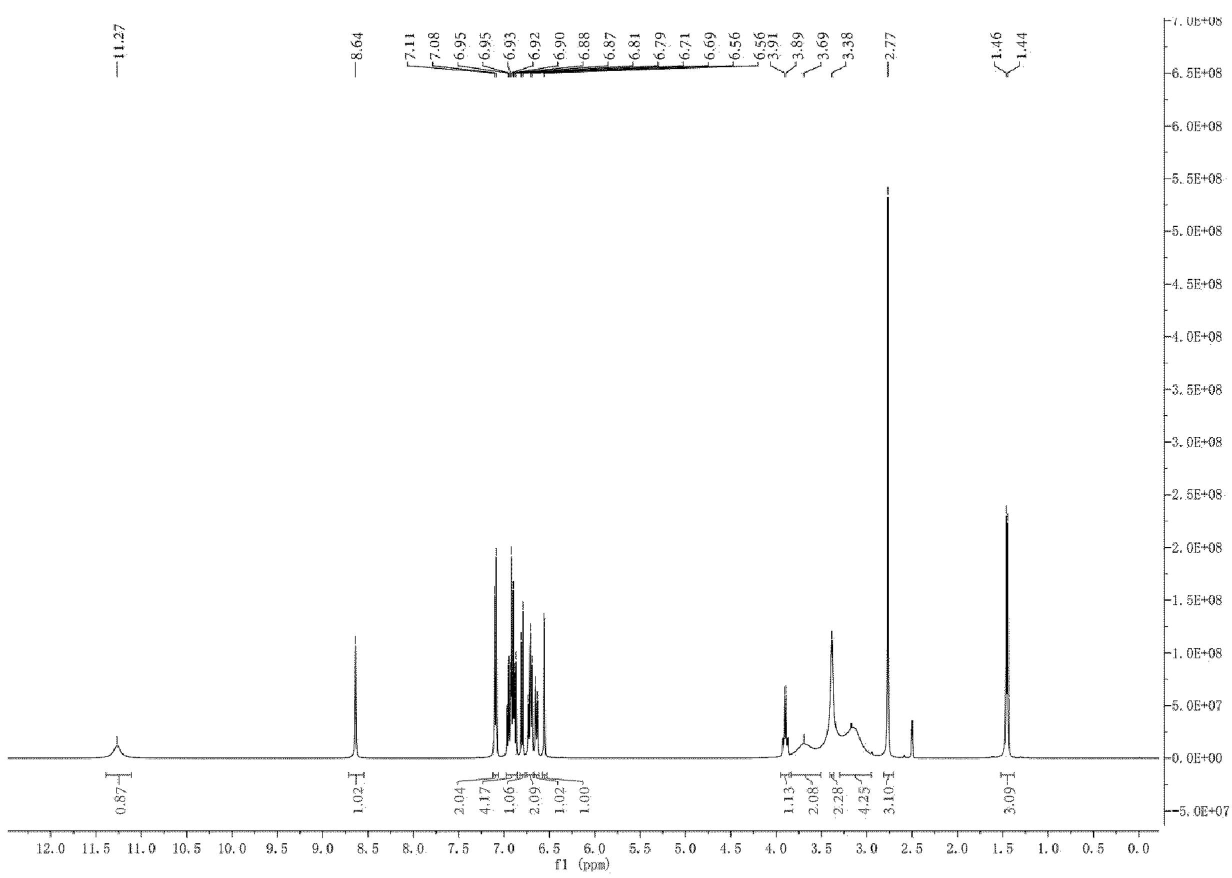
11.27
8.64
7.11
7.08
6.95
6.95
6.93
6.92
6.90
6.88
6.87
6.81
6.79
6.71
6.69
6.56
6.56
3.91
3.89
3.69
3.38
2.77
1.46
1.44
0.87
1.02
2.04
4.17
1.06
2.09
1.02
1.00
1.13
2.08
2.28
4.25
3.10
3.09
f1 (ppm)

FIG. 5

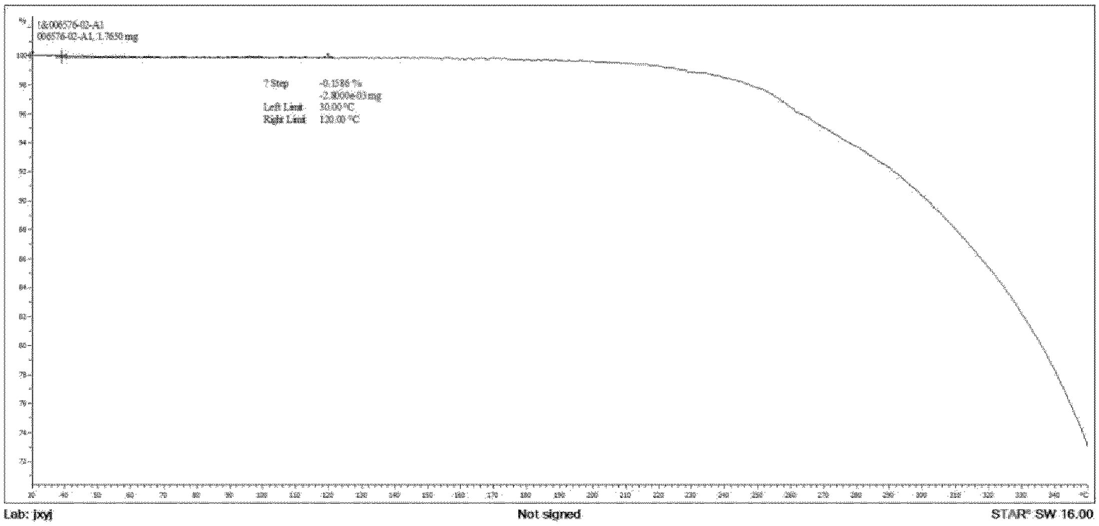
!&006576-02-A1
006576-02-A1, 1.7650 mg
? Step -0.1586 %
-2.8000e-03 mg
Left Limit 30.00 °C
Right Limit 120.00 °C
Lab: jxyj
Not signed
STAR® SW 16.00

FIG. 6

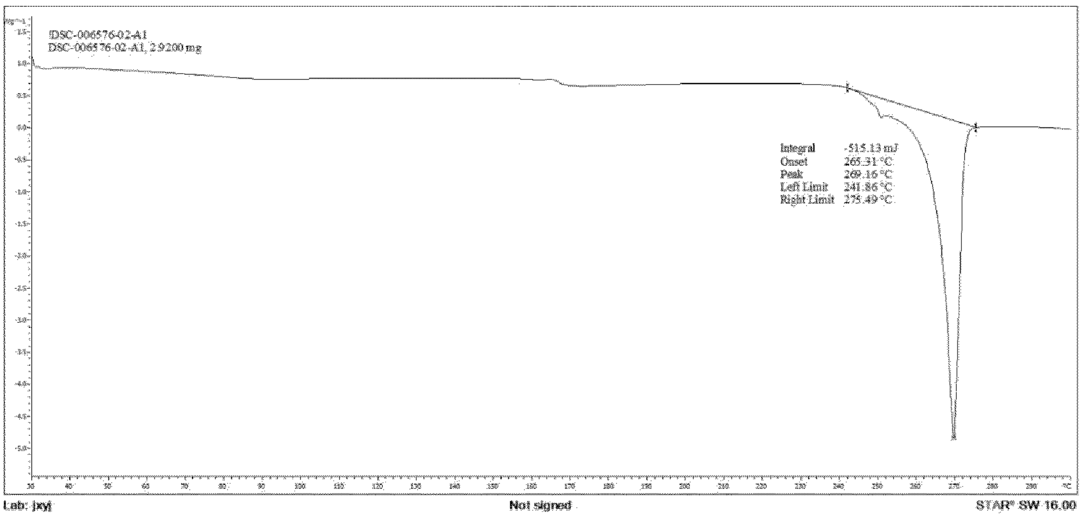
!DSC-006576-02-A1
DSC-006576-02-A1, 2.9200 mg
Integral -515.13 mJ
Onset 265.31 °C
Peak 269.16 °C
Left Limit 241.86 °C
Right Limit 275.49 °C
Lab: jxyj
Not signed
STAR® SW 16.00

FIG. 7

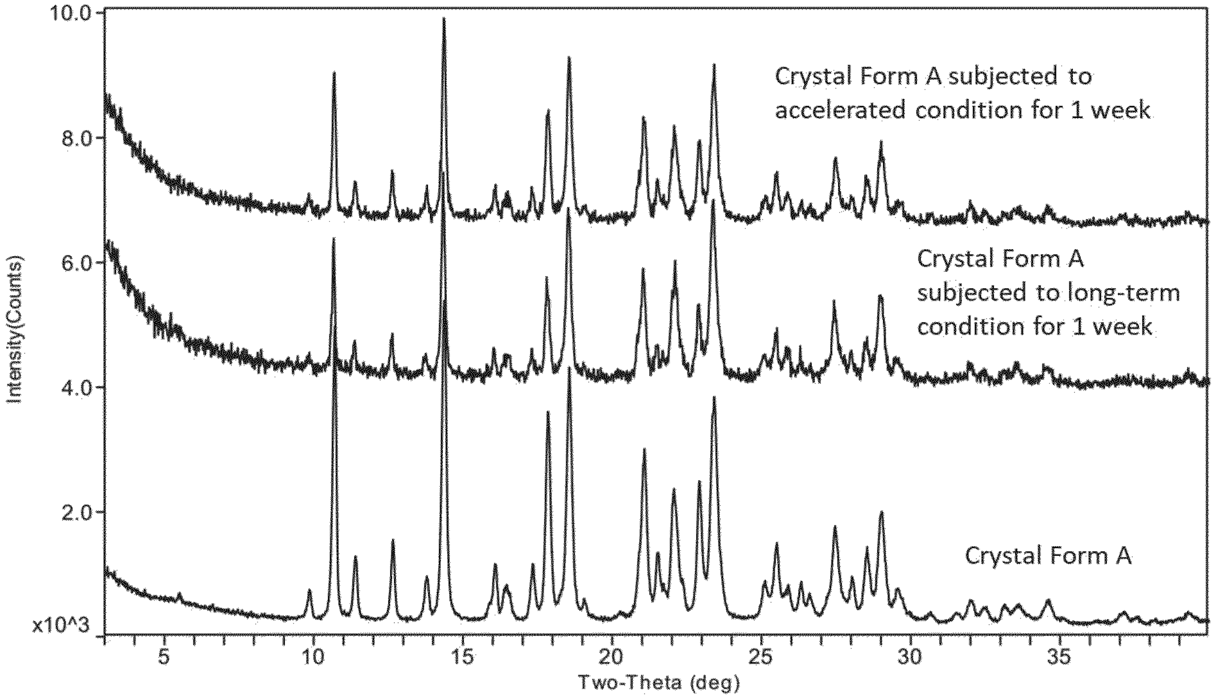
10.0
8.0
6.0
4.0
2.0
x10^3
Intensity(Counts)
5
10
15
20
25
30
35
Two-Theta (deg)
Crystal Form A subjected to accelerated condition for 1 week
Crystal Form A subjected to long-term condition for 1 week
Crystal Form A

FIG. 8

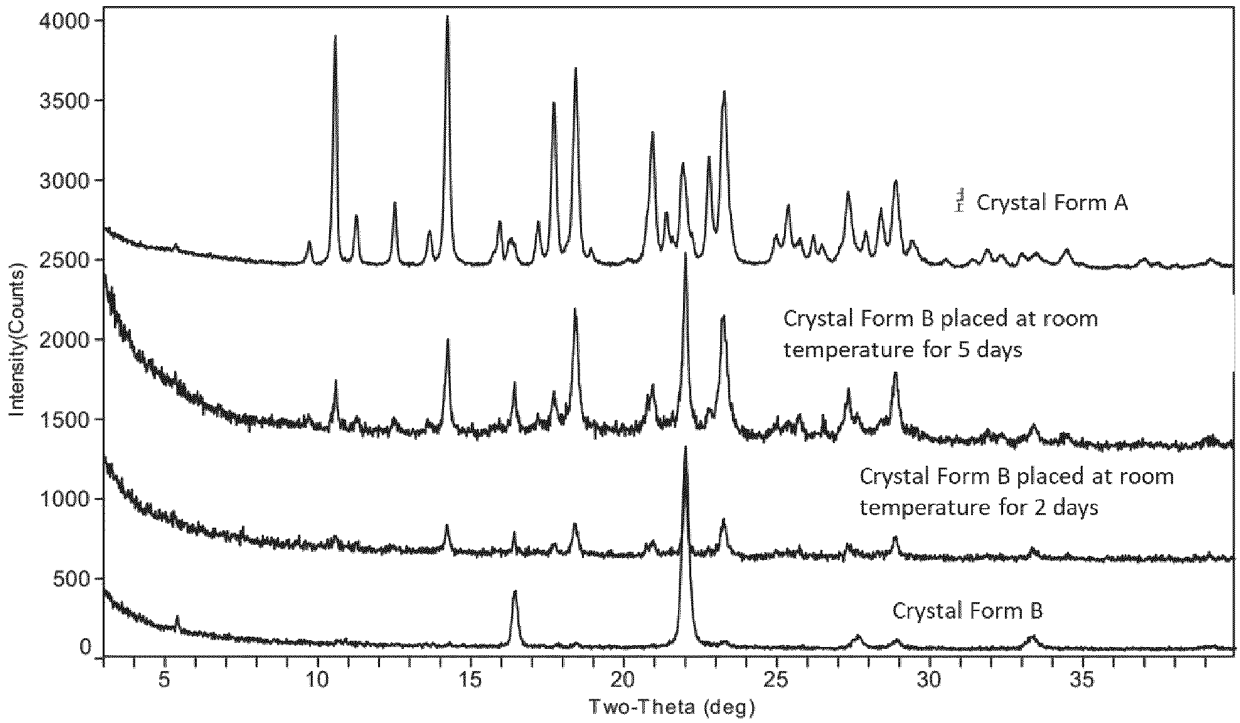

4000
3500
3000
2500
2000
1500
1000
500
0
Intensity(Counts)
5
10
15
20
25
30
35
Two-Theta (deg)
Crystal Form A
Crystal Form B placed at room temperature for 5 days
Crystal Form B placed at room temperature for 2 days
Crystal Form B


FIG. 9

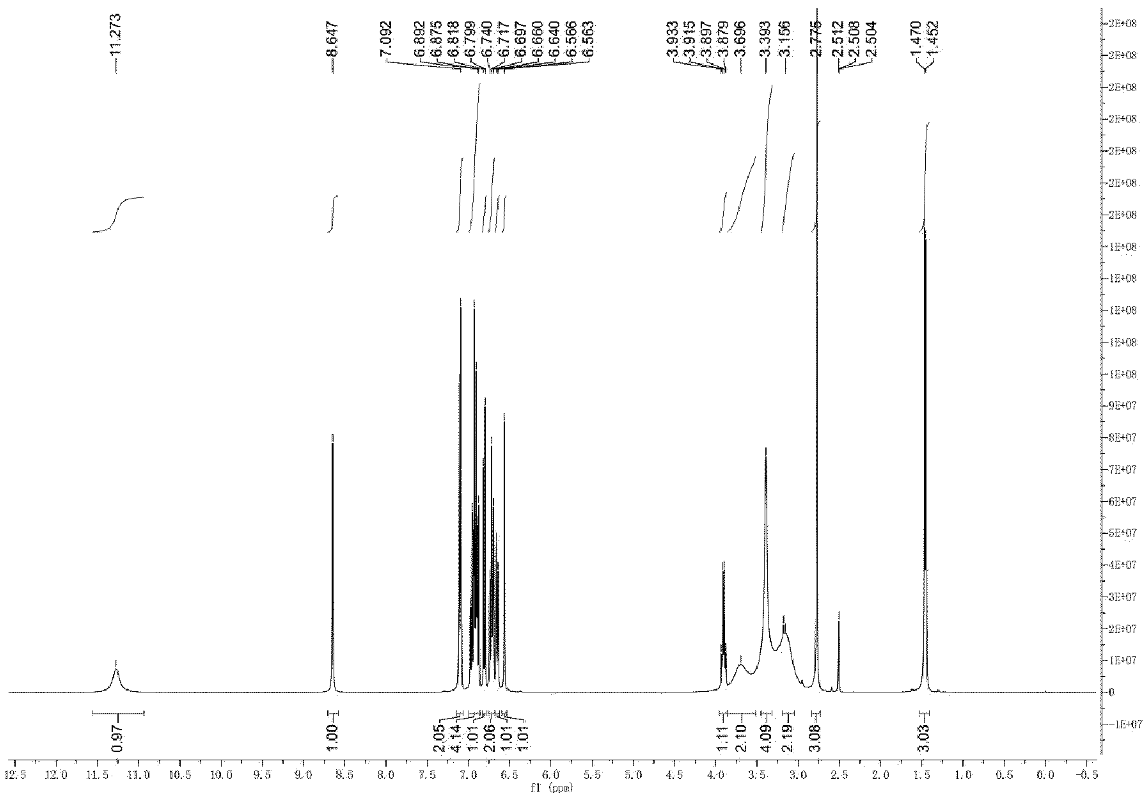

FIG. 10

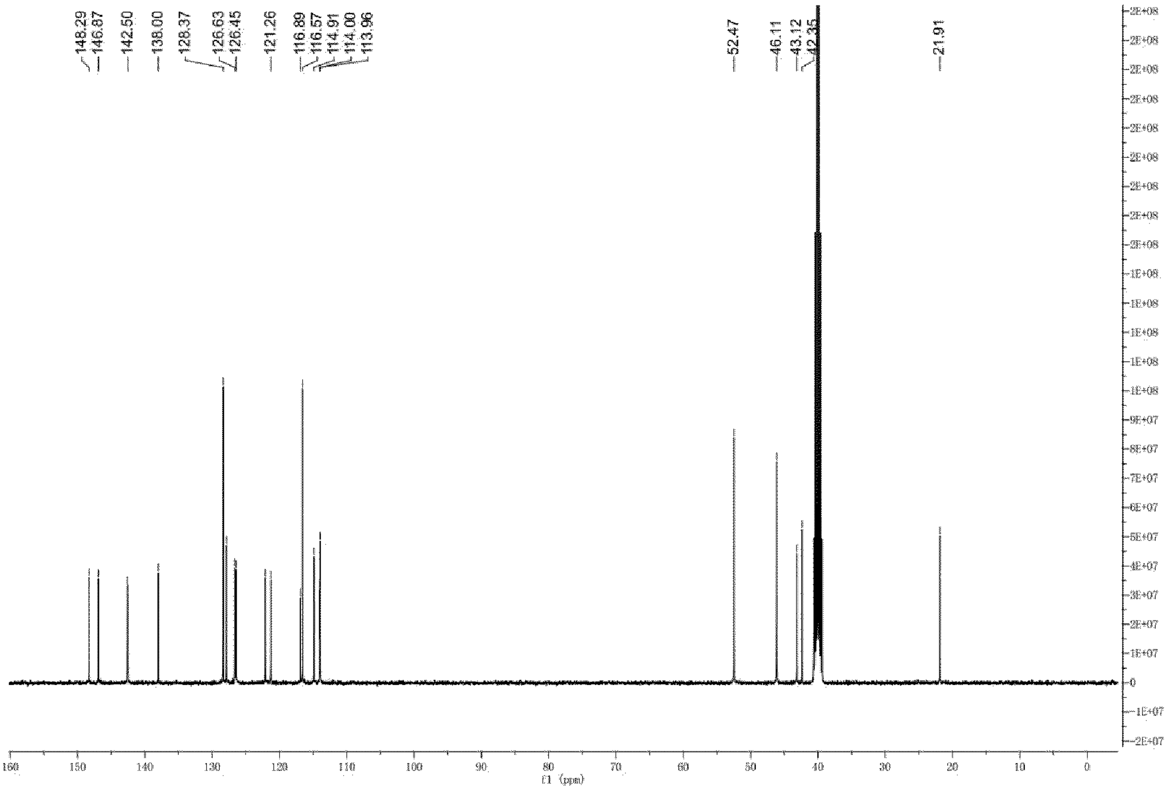
148.29
146.87
142.50
138.00
128.37
126.63
126.45
121.26
116.89
116.57
114.91
114.00
113.96
52.47
46.11
43.12
42.35
21.91
f1 (ppm)

FIG. 11

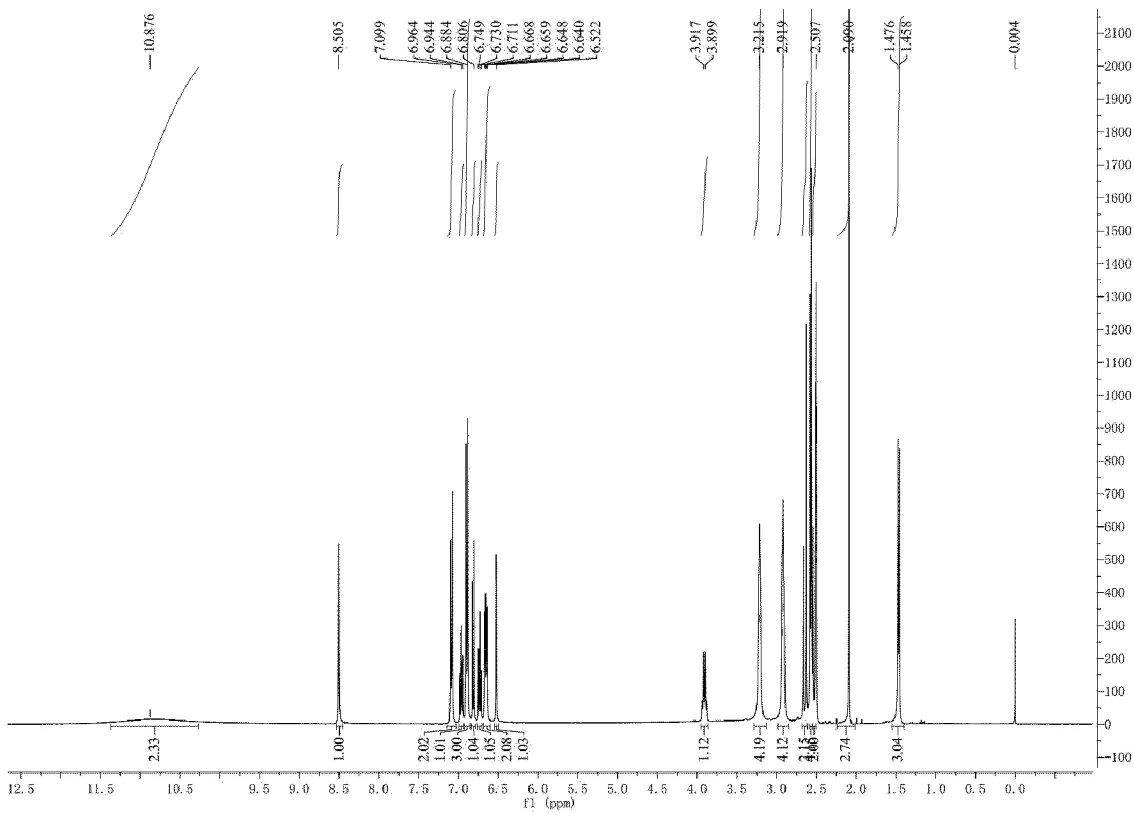

FIG. 12

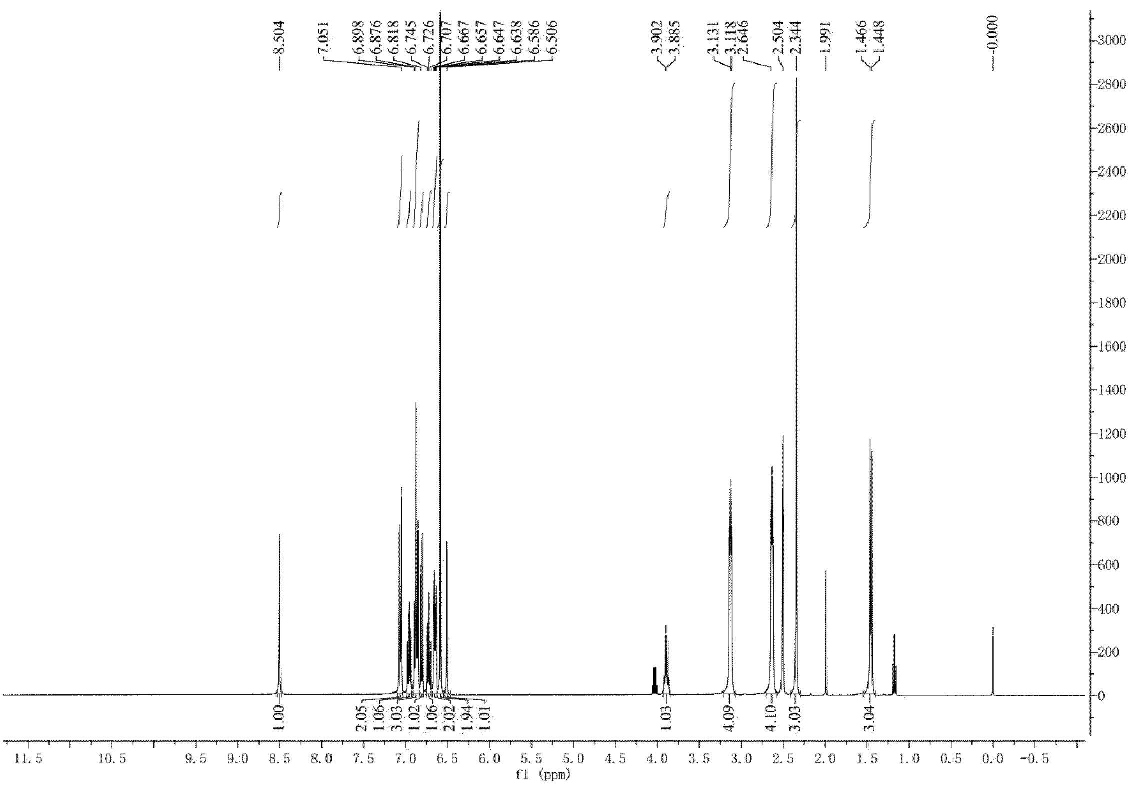

FIG. 13

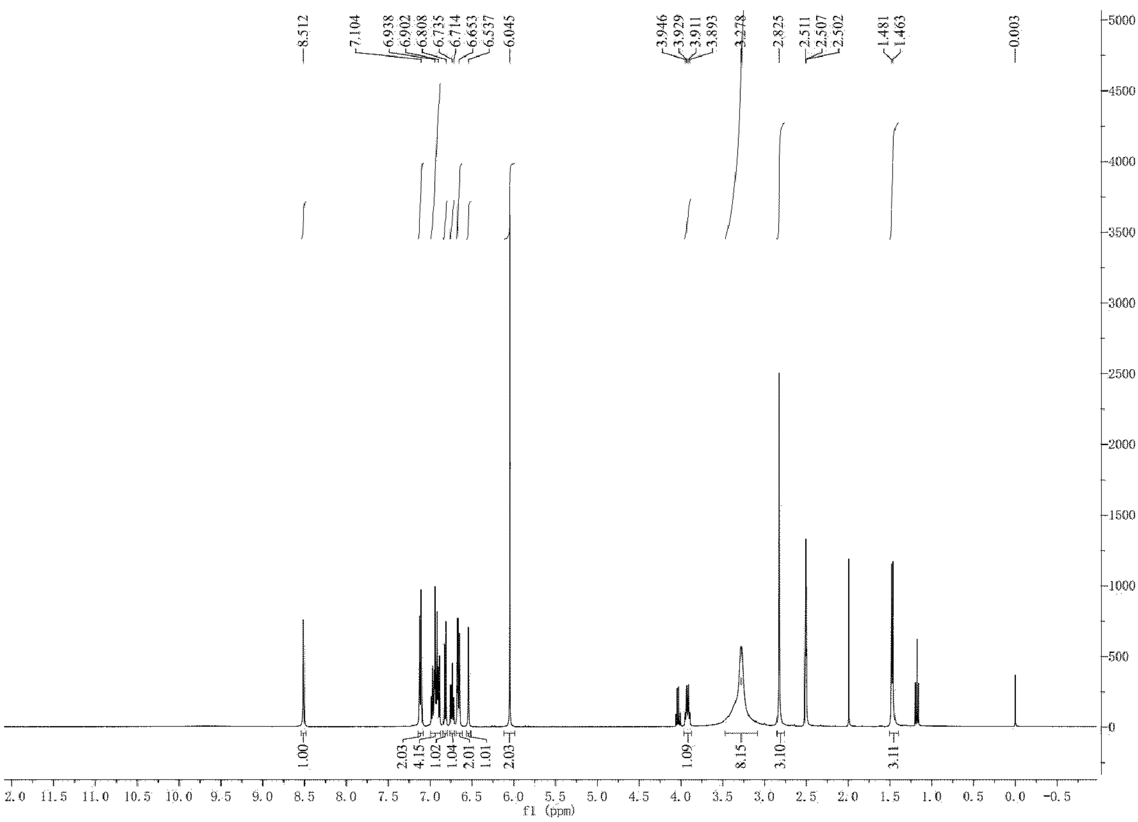
8.512
7.104
6.938
6.902
6.808
6.735
6.714
6.653
6.537
6.045
3.946
3.929
3.911
3.893
3.278
2.825
2.511
2.507
2.502
1.481
1.463
0.003
1.00
2.03
4.15
1.02
1.04
2.01
1.01
2.03
1.09
8.15
3.10
3.11
f1 (ppm)

FIG. 14

**INTERNATIONAL SEARCH REPORT**

International application No.
**PCT/CN2023/073055**

**A. CLASSIFICATION OF SUBJECT MATTER**

C07D279/20(2006.01)i;A61K31/5415(2006.01)i;A61P35/00(2006.01)i;A61P3/00(2006.01)i;A61P9/00(2006.01)i;A61P25/00(2006.01)i

According to International Patent Classification (IPC) or to both national classification and IPC

**B. FIELDS SEARCHED**

Minimum documentation searched (classification system followed by classification symbols)

IPC: C07D,A61K,A61P

Documentation searched other than minimum documentation to the extent that such documents are included in the fields searched

Electronic data base consulted during the international search (name of data base and, where practicable, search terms used)

CNTXT, ENTXTC, CNKI, STN: 成都恒昊创新科技有限公司, 吩噻嗪, 铁死亡抑制剂, phenothiazine, ferroptosis, 结构检索

**C. DOCUMENTS CONSIDERED TO BE RELEVANT**

| Category* | Citation of document, with indication, where appropriate, of the relevant passages | Relevant to claim No. |
|---|---|---|
| X | WO 2019205854 A1 (SICHUAN UNIVERSITY) 31 October 2019 (2019-10-31)<br>see abstract, and claims 1, 5, and 9-10 | 1-18 |
| X | YANG, Wei et al. "Structure-Activity Relationship Studies of Phenothiazine Derivatives as a New Class of Ferroptosis Inhibitors Together with the Therapeutic Effect in an Ischemic Stroke Model"<br>*European Journal of Medicinal Chemistry,*<br>Vol. vol. 209, 18 September 2020 (2020-09-18), 112842<br>see abstract, and table 3 | 1-18 |
| A | CN 111574474 A (CHENGDU HENGHAO INVESTMENT CO., LTD.) 25 August 2020 (2020-08-25)<br>see abstract, and claims 1 and 3 | 1-18 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **01 May 2023** | **09 May 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2023/073055**

| Patent document cited in search report | | | Publication date (day/month/year) | Patent family member(s) | | | Publication date (day/month/year) |
|---|---|---|---|---|---|---|---|
| WO | 2019205854 | A1 | 31 October 2019 | US | 2021040079 | A1 | 11 February 2021 |
| | | | | US | 11440909 | B2 | 13 September 2022 |
| | | | | JP | 2021526157 | A | 30 September 2021 |
| | | | | CA | 3098219 | A1 | 31 October 2019 |
| | | | | EP | 3786156 | A1 | 03 March 2021 |
| | | | | EP | 3786156 | A4 | 23 February 2022 |
| | | | | AU | 2019258078 | A1 | 19 November 2020 |
| CN | 111574474 | A | 25 August 2020 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210108728
- CN 202210108746
- WO 2019205854 A1


### Non-patent literature cited in the description


- **SCOTT J DIXON et al.** *Cell*, 25 May 2012, vol. 149 (5), 1060-72
- **XUEJUN JIANG et al.** *Nat Rev Mol Cell Biol.*, April 2021, vol. 22 (4), 266-282